# EUROPEAN PATENT SPECIFICATION

(11) **EP 1 430 925 B1**
(45) Date of publication and mention of the grant of the patent: **18.05.2011**
(21) Application number: 02800022.2
(22) Date of filing: 30.09.2002
(51) Int. Cl.: A61M 29/00, A61M 25/00

(54) **STENT DELIVERY CATHETER**
STENT-ABGABEKATHETER
CATHETER DE POSE D'ENDOPROTHESE

(30) Priority: 28.09.2001 JP 2001303753; 28.09.2001 JP 2001303754; 28.09.2001 JP 2001303755
(43) Date of publication of application: 23.06.2004
(73) Proprietor: KANEKA CORPORATION, Osaka-shi, Osaka 530-0005 (JP)
(72) Inventor: NISHIDE, Takuji, Settsu-shi, Osaka 566-0072 (JP); MIKI, Shogo, Chigasaki-shi, Kanagawa 253-0084 (JP)
(74) Representative: Gille Hrabal Struck Neidlein Prop Roos
(86) International application number: PCT/JP2002/010153
(87) International publication number: WO 2003/028795

(56) References cited:
- EP-A1- 0 982 010
- WO-A-00/45740
- WO-A-00/74597
- WO-A-01/34240
- JP-A- 9 503 411
- JP-A- 2000 217 924
- US-A- 5 254 091
- US-A- 5 328 472
- US-A- 5 968 069
- US-A1- 2001 000 350
- US-A1- 2001 007 082
- US-B1- 6 293 959

## Description

### Technical Field

The present invention relates generally to delivery catheters for introducing and placing stents for expanding narrowed areas of blood vessels, esophagi, tracheae, urethrae, bile ducts, etc. In particular, it relates to a delivery catheter for introducing and placing a stent to a narrowed area of a coronary artery.

### Background Art

Stents, which are left in narrowed areas of vessels such as blood vessels, esophagi, tracheae, urethrae, and bile ducts, are widely used devices for efficiently maintaining lumina. A stent is folded to be introduced into a vessel and is placed at a predetermined narrowed area. Subsequently, the stent is deployed to a predetermined size and left in that location.

Stents are roughly categorized into two types according to the mechanism for expanding stents to predetermined sizes. One type is of a self-expandable stent variety. Stents of this type are composed of shape-memory alloys and do not require mechanical stent expansion. The other type is of a balloon-expandable stent variety. Stents of this type require mechanical stent expansion and are generally deployed by utilizing known balloon catheters for expanding vessels, namely, arteries and veins.

Stents of the balloon-expandable type do not themselves have expansion functions. In general, in order to place stents of this type to predetermined narrowed areas, stents attached on balloons of balloon catheters are first introduced to predetermined narrowed areas. Subsequently, balloons are inflated to plastically deform the stents by the expansive force of the balloons so that the stents tightly adhere onto inner walls of the narrowed areas.

In order to place a balloon-expandable stent according to the above-described process, insertion of a balloon catheter having a stent mounted on a balloon to a narrowed area is necessary. During the insertion, the stent on the balloon may move and fall off from the balloon catheter. A typical balloon of a balloon catheter has a straight tubular portion that can expand into a cylindrical shape and two frustoconical tapered portions respectively disposed at the front end and the back end of the straight tubular portion. Since the stent is generally mounted on the outer wall of the straight tubular portion, the stent on the balloon may move to the front or end portion during insertion even if the stent is prevented from falling off. In such a case, one end of the stent is positioned on the outer wall of the taper portion of the balloon. Since this portion of the balloon expands into a tapered shape, the expansion of the stent at this portion becomes insufficient. As a result, it is highly likely that restenosis would occur at the narrowed area.

In order to overcome such drawbacks, related art discloses techniques for preventing falling or displacement of stents of balloon catheters used in delivering balloon-expandable stents.

Japanese Unexamined Patent Application Publication No. 8-164210 discloses a vascular supporting device having means for encapsulating a stent. According to this prior art, encapsulation is achieved by applying heat and pressure to the balloon so that the balloon can expand around a folded stent and by subsequently cooling the balloon.

However, mechanical or thermal damage may be inflicted upon the balloon during the process of heating, pressurizing, and cooling the balloon. The compression strength may decrease and pinholes may be generated as a result.

Japanese Unexamined Patent Application Publication No. 9-276414 discloses a stent delivery system having stent retention means for preventing a stent on a balloon from moving. In particular, it discloses a technique of forming a layer having a high friction coefficient on the outer wall of the balloon and a technique of forming a smaller diameter portion (saddle or sheet) in an inner tubular member inside the balloon and disposing a stent on the balloon outer wall corresponding to that sheet portion.

Formation of the high friction coefficient layer on the outer wall of the balloon complicates the manufacturing process and thus produces cost problems. In forming the smaller diameter portion (saddle or sheet) in the inner tubular member inside the balloon and disposing the stent on the balloon outer wall corresponding to the sheet portion, the inner diameter of the inner tubular member must be large enough to allow insertion of the guidewire. Thus, it is obvious that the thickness of the inner tubular member at the sheet portion is smaller than other portions. Accordingly, the sheet portion of the inner tubular member may kink in the course of delivering the stent to the narrowed area, thereby significantly degrading maneuverability.

Japanese Unexamined Patent Application Publication No. 11-128366 discloses a catheter structure in which two collars disposed on the external periphery of a portion located inside the balloon oppose each other in parallel with a distance therebetween. In this structure, a tubular adaptor is disposed on the external periphery of the portion between the collars and the balloon is wound around the collars and the tubular adaptor so as to form a mechanism for preventing a stent, which is mounted to surround the balloon, from moving in the axis direction of the stent.

According to this prior art, the possibility of degradation in catheter maneuverability due to kinking of the inner tubular member experienced in the related art disclosed in Japanese Unexamined Patent Application Publication No. 9-276414 may be smaller. However, since the two collars are fixed onto portions inside the balloon, the flexibility of the balloon portion is significantly decreased. Accordingly, a stent cannot be delivered to a tortuous narrowed area.

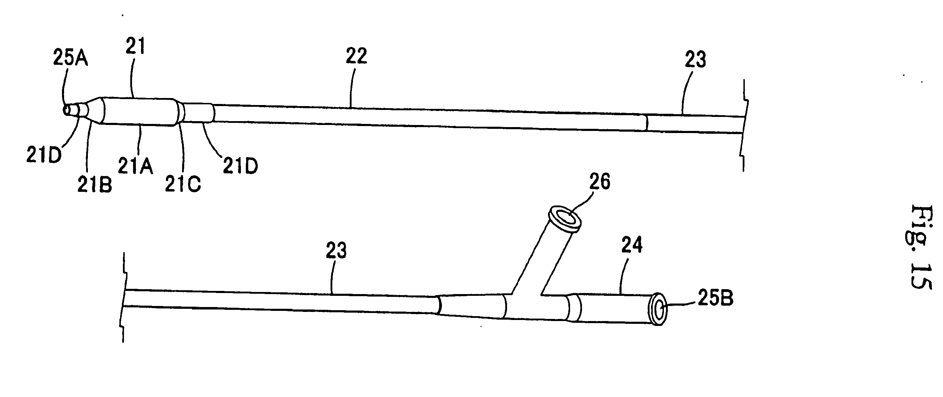

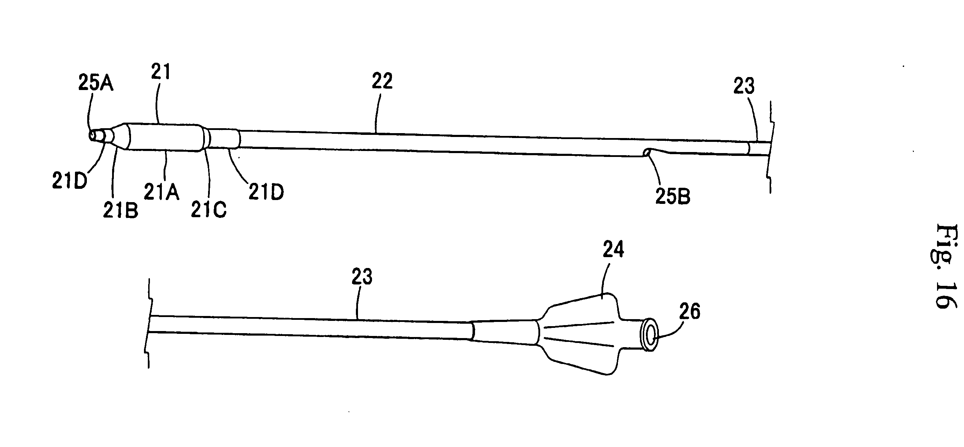

Figs. 15 and 16 show balloon catheters frequently used with balloon-expandable stents. Each catheter has a distal-end shaft 22 having a balloon 21 attached at the tip and a proximal-end shaft 23 joined to a hub 24 having a port for supplying pressure fluid for controlling the internal pressure of the balloon. This type of catheter is roughly categorized into two types according to the length of a guidewire lumen 25.

One category is of an over-the-wire (OTW) type shown in Fig. 15. The guidewire lumen 25 is provided over the entire length of the balloon catheter. A proximal-end opening 25B of the guidewire lumen 25 is formed in the hub 24, and a distal-end opening 25A of the guidewire lumen 25 is formed in the tip of the balloon 21 or beyond the tip of the balloon 21. The other category is of a rapid exchange (RX) type shown in Fig. 16. As shown in the drawing, the guidewire lumen 25 is provided only at a portion near the distal end of the balloon catheter, and the proximal-end opening 25B of the guidewire lumen 25 is formed in the distal-end shaft 22. Since the guidewire lumen lies over the entire length of the OTW-type balloon catheter, this type of catheter is frequently used to insert the guidewire to the site of a lesion, such as severe stenosis, in particular chronic total occlusion (CTO), in which the insertion of the guidewire to the site of the lesion is difficult. A drawback of the balloon catheter of this type is that the operation of removing the balloon catheter without removing the guidewire from the site of the lesion is complicated. In particular, a special device or special operation, such as mounting a replacement extension wire, is necessary to remove the balloon catheter while leaving the guidewire at the lesion.

On the other hand, according to the RX type, the guidewire lumen 25 is provided only in the front portion of the balloon catheter. Thus, the balloon catheter can be easily removed, replaced, or inserted while leaving the guidewire at the lesion. Not only the maneuverability is excellent, but also operation time is shortened. Moreover, the number of the required devices can be decreased. This invention is directed to a balloon catheter of the RX type. Various technologies for improving the maneuverability are known.

Japanese Unexamined Patent Application Publication No. 5-28634 discloses a balloon-expandable catheter of the RX type in which an opening of a guidewire lumen is formed in a region where an intermediate portion is joined to a base portion, and in which the entire catheter is continuously supported in the longitudinal direction over the entire length when the guidewire is accommodated in the guidewire lumen.

In this related art, good maneuverability is achieved since the catheter is continuously supported in the longitudinal direction when the guidewire is accommodated. However, the rigidity of the catheter itself changes discontinuously in the longitudinal direction. Accordingly, when the catheter is inserted from the outside the body along the guidewire, breakage of the catheter frequently occurs at the region where the intermediate portion and the base portion are joined, resulting in poor maneuverability.

PCT Japanese Translation Patent Publication No. 6-507105 discloses a vascular catheter comprising a main shaft constituted from a metal tube; a balloon; a plastic shaft portion between the main shaft and the balloon; an intermediate member attached to the main shaft, extending into the plastic shaft portion in the proximal-end direction, and less rigid than the main shaft portion; and a guidewire lumen, wherein the guidewire inlet is located a particular distance away from the proximal end of the main shaft portion in the proximal-end direction.

This related art achieves a catheter with good push and track ability and improved maneuverability in inserting the catheter along the guidewire from outside the body. However, this related art has a problem of manufacturing. In particular, an additional step is required to join the intermediate member, which is less rigid than the main shaft portion, to the main shaft. For example, the step of brazing or laser bonding is necessary, thereby resulting in complication of the process and an increase in the manufacturing cost.

PCT Japanese Translation Patent Publication No. 9-503411 discloses an expandable catheter having a stylet for enhancing the pressure resistance and the transferability (push performance) in the axial direction of the catheter shaft.

According to this related art, the stylet improves the transferability (push performance) of the catheter shaft in the axial direction while enhancing the maneuverability in inserting the catheter along the guidewire from outside the body. However, since the base end of the stylet is configured to be located at the hub member including the base portion of the catheter shaft, the stylet lies substantially over the entire inflation lumen, thereby degrading the responsiveness for expanding and contracting the balloon.

WO 01/34 240 A only discloses a stent delivery system with stent retaining means that is affixed to an inner tube.

### Disclosure of Invention

In view of the above, an object of the present invention is to provide a stent delivery catheter that can place a stent in a tortuous narrowed area with good maneuverability while preventing falling or displacement of the stent during the insertion of the stent-mounted balloon catheter to the narrowed area.

In order to achieve the object, the invention provides a stent delivery catheter for delivering a stent for treating stenosis in a body to a narrowed area, the stent delivery catheter including a distal end and a proximal end, in which the proximal end includes a hub having a port for supplying a pressure fluid, and the distal end includes a collapsible balloon in a collapsed state and the stent in an undeployed state, the stent being mounted on the outer surface of the collapsed balloon, the balloon having frustoconical tapered segments and a cylindrical straight tubular segment. The stent delivery catheter further includes an inner tube for defining a guidewire lumen extending into the interior of the balloon; and displacement prevention mechanisms for preventing the stent from moving in the longitudinal direction of the stent delivery catheter, the displacement prevention mechanisms being affixed to the inner surface of the balloon only.

Preferably, the displacement prevention mechanisms are disposed at a distal end and a proximal end of the balloon and are preferably tubular.

Preferably, the displacement prevention mechanisms lie only in the tapered segments of the balloon and do not extend to the interior of the balloon. More preferably, the relationship 1 ≤ (D2/D1), and most preferably the relationship 1 ≤ (D2/D1) ≤ 2 are satisfied, wherein D1 is the outer diameter of the displacement prevention mechanism at the portion affixed to the balloon, and D2 is the outer diameter of the displacement prevention mechanism at the portion extending into the interior of the balloon.

Each displacement prevention mechanism is preferably composed of a material that can be melt-bonded with the balloon. More preferably, the displacement prevention mechanism is composed of polyamide or a polyamide elastomer, and the balloon is composed of a polyamide elastomer or a blend material containing polyamide elastomers. Alternatively, the displacement prevention mechanism may be composed of polyester or a polyester elastomer, and the balloon may be composed of a polyester elastomer or a blend material containing polyester elastomers.

Preferably, a radiopaque marker is mounted on the displacement prevention mechanism.

The Inventions set forth in Japanese Unexamined Patent Application Publication Nos. 8-164210 and 9-276414 are both effective for retaining the stent; however, they require additional steps in the manufacturing process and inevitably complicate the process. Moreover, the invention set forth in Japanese Unexamined Patent Application Publication No. 11-128366 require joining of two.collars and a tubular adaptor on the external periphery of the portion inside the balloon and thus inevitably complicates the manufacturing process and increases the manufacturing cost.

Thus, in order to overcome these drawbacks, it is also described a method to provide a stent delivery catheter that can prevent a stent from falling or moving in the course of inserting the catheter to a narrowed area, in which no additional components are necessary to prevent displacement, and the number of components or manufacturing steps does not increase. Thus, the manufacturing process can be streamlined without complication.

This method describes a stent delivery catheter for delivering a stent for treating stenosis in a body to a narrowed area, the stent delivery catheter including a distal end and a proximal end, in which the proximal end includes a hub having a port for supplying a pressure fluid, and the distal end includes a collapsible balloon in a collapsed state and the stent in an undeployed state, the stent being mounted on the outer surface of the collapsed balloon, the balloon having a cylindrical straight tubular segment, a frustoconical distal-end tapered segment disposed at a distal end of the balloon and a frustoconical proximal-end tapered segment disposed at a proximal end of the balloon. In this structure, the thickness T1 of a near-center portion of the distal-end tapered segment and the thickness T2 of a near-center portion of the straight tubular segment satisfy the relationship 1.3 s (T1/T2) ≤ 2.5, and the thickness T3 of a near-center portion of the proximal-end tapered segment and the thickness T2 of the near-center portion of the straight tubular segment satisfy the relationship 1.3 s (T3/T2) ≤ 2.5. Moreover, the stent is prevented from moving in the axis direction of the stent catheter due to the presence of the distal-end tapered segment and the proximal-end tapered segment of the collapsed balloon.

The thickness T1 of the near-center portion of the distal-end tapered segment and the thickness T2 of the near-center portion of the straight tubular member preferably satisfy the relationship 1.6 ≤ (T1/T2) ≤ 2.5, and the thickness T3 of the near-center portion of the proximal-end tapered segment and the thickness T2 of the near-center portion of the straight tubular member satisfy the relationship 1.6 s (T3/T2) ≤ 2.5.

Another object of the present invention is to provide a RX-type balloon catheter having improved maneuverability in inserting the balloon catheter along the guidewire from outside the body and enhanced responsiveness for balloon expansion and contraction without complicating the process and increasing the manufacturing cost.

Through investigations were conducted to overcome the problems of balloon catheters for balloon-expandable stents. In view of the above, an example provides a balloon catheter including a proximal-end shaft including a metal tube having a distal-end segment and a proximal-end segment; a distal-end shaft comprising a resin tube; a balloon expandable and collapsible by adjusting the pressure of the interior; a guidewire lumen that can accommodate a guidewire, the guidewire lumen having a distal-end opening and a proximal-end opening; and an inflation lumen for supplying a pressure fluid into the balloon, the guidewire lumen extending in the balloon catheter distal-end direction over the interior of the balloon so as to protrude from the balloon, the distal-end opening of the guidewire lumen being formed in the front-most tip of the balloon catheter, the proximal-end opening of the guidewire lumen being formed in the distal-end shaft, the distal end of the proximal-end shaft being joined with the proximal end of the distal-end shaft, and the distal end of the distal-end shaft being joined with the balloon. In this structure, a core wire for adjusting the flexibility of the distal-end shaft is disposed inside the inflation lumen so that the portion extending from the proximal-end opening of the distal-end shaft to the proximal end of the distal-end shaft is harder than the portion extended from the proximal-end opening of the distal-end shaft to the distal end of the distal-end shaft and is softer than the proximal-end shaft. Moreover, the core wire is affixed to the distal-end shaft only at the region near the proximal-end opening of the guidewire lumen. Preferably, the portion of the core wire in the region affixed to the distal-end shaft is wrapped with a resin layer that can melt-bond with the inner wall of the distal-end shaft for defining the inflation lumen.

More preferably, the distal end of the core wire is located at a position a predetermined distance away from the proximal-end opening of the guidewire lumen in the distal-end direction, and the proximal end of the core wire is located inside the proximal-end shaft but does not extend up to the proximal end of the proximal-end shaft.

Preferably, the outer diameter of at least part of the core wire in the region that extends in the proximal-end direction from the proximal-end opening of the guidewire lumen in the distal-end shaft decreases toward the distal end of the core wire to form a tapered shape.

### Brief Description of the Drawings

Fig. 1 is a schematic perspective view of a typical over-the-wire balloon catheter.
Fig. 2 is a schematic perspective view of a typical rapid-exchange balloon catheter.
Fig. 3 is a schematic cross-sectional view showing a coaxial structure of a typical balloon catheter.
Fig. 4 is a cross-sectional view taken along line A-A' of Fig. 3.
Fig. 5 is a schematic cross-sectional view showing a biaxial structure of a typical balloon catheter.
Fig. 6 is a cross-sectional view taken along line B-B' of Fig. 5.
Fig. 7 is a schematic cross-sectional view of a stent delivery catheter according to the invention, in which displacement prevention mechanisms are disposed at distal and proximal ends of the balloon.
Fig. 8 is a schematic cross-sectional view of the stent delivery catheter of the invention, in which the displacement prevention mechanism at the proximal end of the balloon is an outer tube.
Fig. 9 is a schematic cross-sectional view of the tent delivery catheter of the invention, in which the displacement prevention mechanisms are disposed at the distal and proximal ends of the balloon and the outer diameters of the portions of the displacement prevention mechanisms are larger than the outer diameters of the affixed portions.
Fig. 10 is a schematic cross-sectional view of another embodiment of that shown in Fig. 9.
Fig. 11 is a schematic cross-sectional view of the stent delivery catheter of this invention, in which the displacement prevention mechanisms are disposed at the distal and proximal ends of the balloon and radiopaque markers are provided on portions that lie inside the balloon.
Fig. 12 is a schematic perspective view showing the balloon shown in Fig. 3 in a collapsed state and the stent mounted on the balloon.
Fig. 13 is a schematic perspective view showing the balloon shown in Fig. 7 in a collapsed state and the stent mounted on the balloon.
Fig. 14 is a schematic view of a system used to evaluate body insertion characteristics.
Fig. 15 is a schematic perspective view of a typical over-the-wire (OTW) balloon catheter.
Fig. 16 is a schematic perspective view of a typical rapid-exchange (RX) balloon catheter.
Fig. 17 is a partial schematic cross-sectional view showing an RX balloon catheter, the cross-sectional view showing a longitudinal cross-section of a coaxial structure of a distal-end segment of a distal-end shaft.
Fig. 18 is a cross-sectional view taken along line C-C' of Fig. 17.
Fig. 19 is a cross-sectional view taken along line D-D' of Fig. 17.
Fig. 20 is a cross-sectional view taken along line E-E' of Fig. 17.
Fig. 21 is a cross-sectional view taken along line F-F' of Fig. 17.
Fig. 22 is a partial schematic cross-sectional view showing an RX balloon catheter, the cross-sectional view showing a longitudinal cross-section of a biaxial structure of the distal-end segment of the distal-end shaft.
Fig. 23 is a cross-sectional view taken along line G-G' of Fig. 22.
Fig. 24 is a cross-sectional view taken along line H-H' of Fig. 22.
Fig. 25 is a cross-sectional view taken along line I-I' of Fig. 22.
Fig. 26 is a cross-sectional view taken along line J-J' of Fig. 22.
Fig. 27 is a schematic perspective view showing one core wire.

### Best Mode for Carrying Out the Invention

Various embodiments of a stent delivery catheter according to the invention will now be described in detail with reference to the drawings.

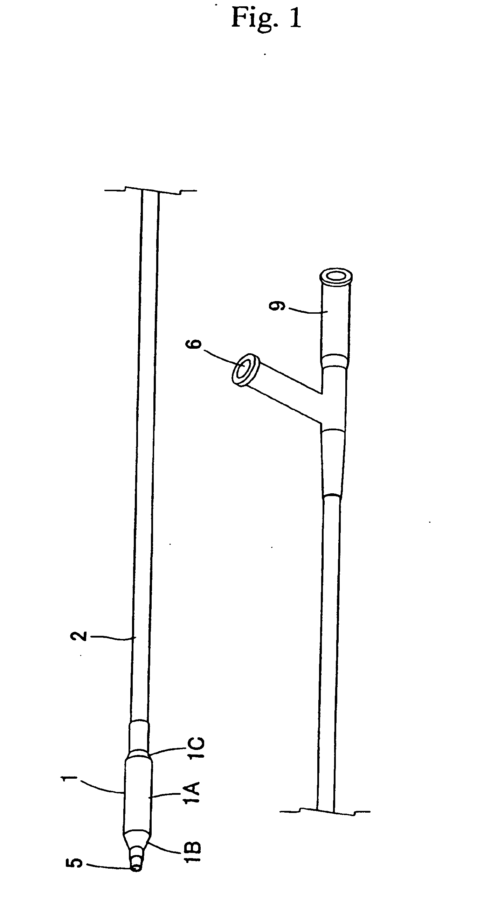

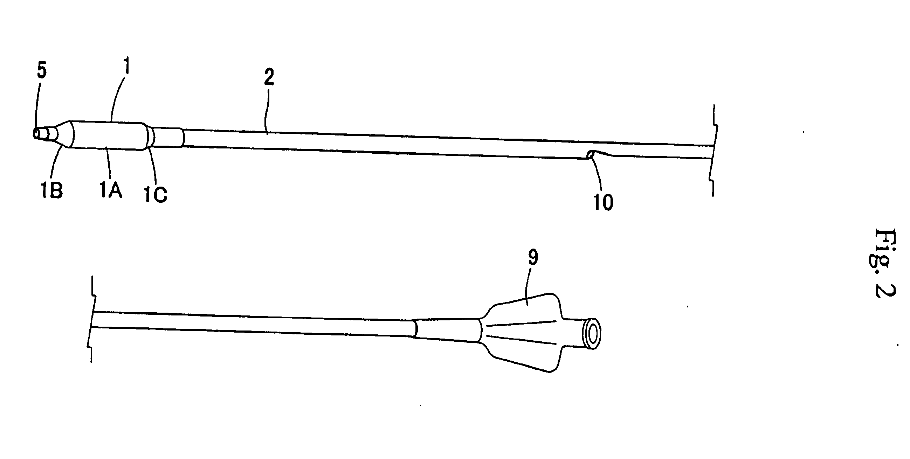

The stent delivery catheter of the present invention may be of the over-the-wire (OTW) type shown in Fig. 1 having a guidewire lumen 5 over the entire length of the catheter or may be of the rapid exchange (RX) type shown in Fig. 2 having the guidewire lumen 5 only at the distal-end portion of the catheter.

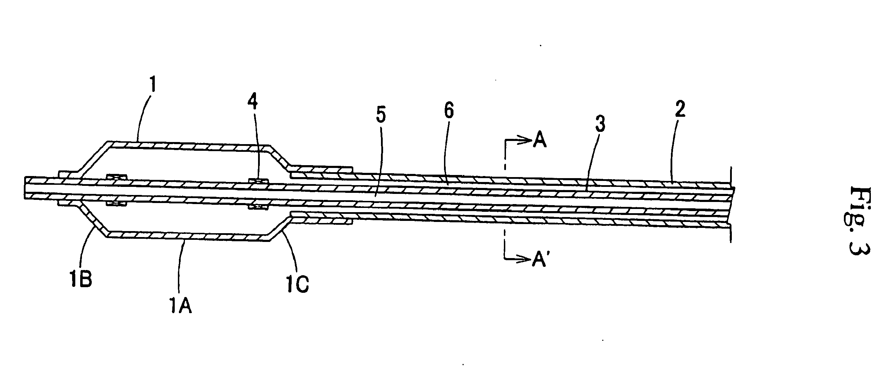

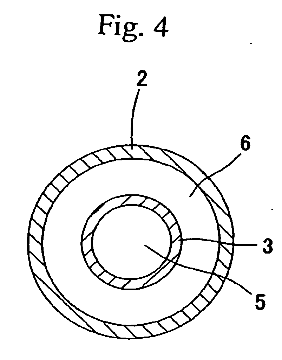

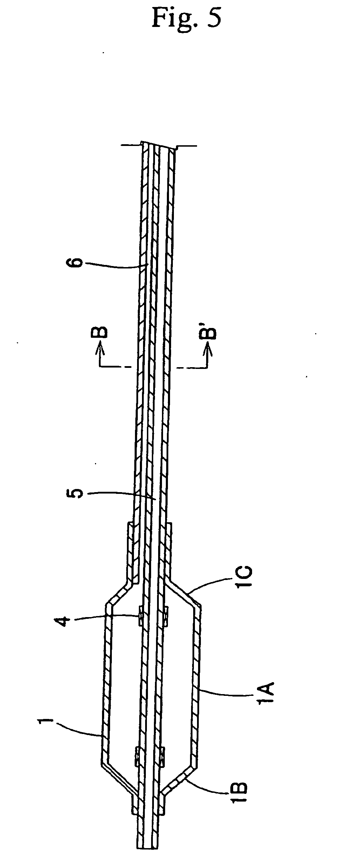

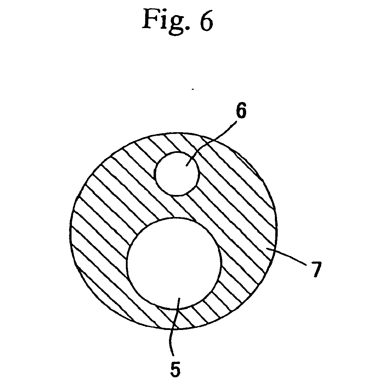

The stent delivery catheter may have any structure as long as the guidewire lumen 5 and an inflation lumen 6 are provided. As shown in Figs. 3 and 4, the stent delivery catheter may be of a co-axial type in which an outer tube 2 and an inner tube 3 are coaxially provided, the guidewire lumen 5 being inside the inner tube and the inflation lumen 6 being defined by the inner wall of the outer tube and the outer wall of the inner tube. Alternatively, as shown in Figs. 5 and 6, the stent delivery catheter may be of a biaxial type having a dual lumen tube 7 that includes the guidewire lumen 5 and the inflation lumen 6 extending in parallel with each other. The effect and advantages of the present invention will not be limited by employing these structures.

A balloon 1 of the present invention preferably has a cylindrical straight tubular segment 1A, a frustoconical tapered segment 1B disposed at the distal end of the straight tubular segment, and a frustoconical tapered segment 1C disposed at the proximal end of the straight tubular segment. The taper angles of the tapered segments 1B and 1C are not limited. Any angle fit for the intended use may be selected.

The present invention will now be described in detail using a stent delivery catheter of a coaxial type as an example.

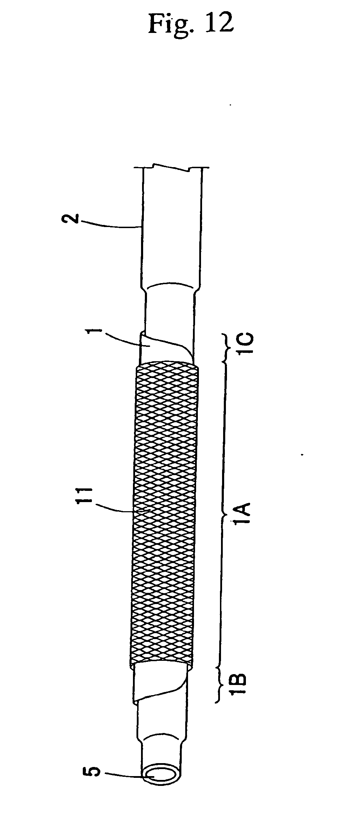

The balloon 1 is collapsible. The balloon 1 can be collapsed to an elongated shape by reducing the pressure inside the balloon so as to fold the balloon along the inner tube 3. A stent in an undeployed state is mounted on the outer wall of the collapsed balloon 1, preferably on the outer wall of the straight tubular segment 1A, so that the stent can be inserted to a human body using the balloon catheter. However, if the undeployed stent 11 (Fig. 12) is mounted on the collapsed balloon 1 of a stent delivery catheter having no displacement prevention mechanism 8 (Fig. 1), the outer diameter of the undeployed stent 11 becomes larger than the outer diameters of the straight tubular segment 1A and the tapered segments 1B and 1C of the collapsed balloon 1. Accordingly, the stent 11 may move on the balloon 1 or may even fall off from the catheter due to the friction with a hemostatic valve or guide catheter that would occur during the insertion of the stent 11 to the human body.

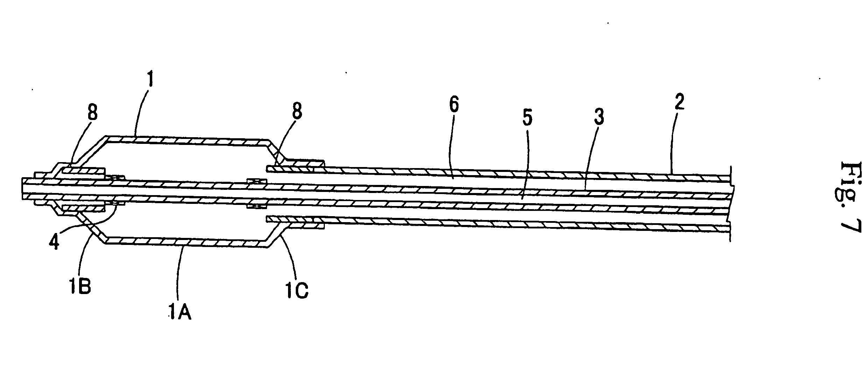

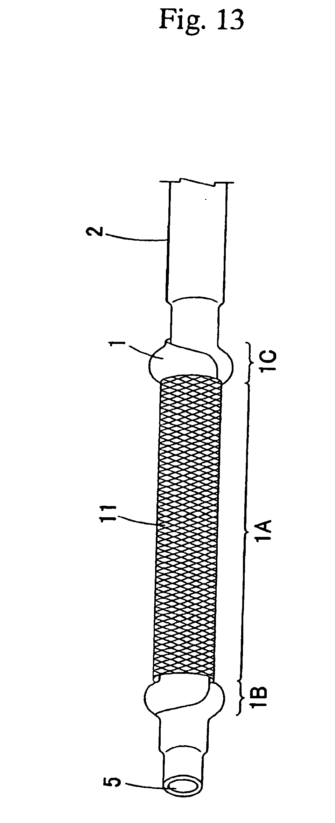

On the other hand, if the undeployed stent 11 is mounted (Fig. 13) on the collapsed balloon 1 of the stent delivery catheter with the displacement prevention mechanisms 8 in accordance with the present invention (Fig. 7), the outer diameters of the tapered segments 1B and 1C of the collapsed balloon 1 becomes larger than the outer diameter of the undeployed stent 11 due to the presence of the displacement prevention mechanisms 8. As a result, the stent 11 can be prevented from moving on the surface of the balloon 1 or falling off from the catheter.

As shown in Fig. 7, the displacement prevention mechanism 8 is preferably disposed at each of the distal-end portion and the proximal-end portion of the balloon 1. When the displacement prevention mechanisms 8 are provided at the distal- and proximal-end portions of the balloon 1, movement of the stent 11 toward the distal-end direction and the proximal-end direction of the stent delivery catheter can be effectively prevented during inserting the stent 11 to a narrowed area. Moreover, falling of the stent, which is highly dangerous, can be prevented.

Each displacement prevention mechanism 8 is preferably fixed onto the inner wall of the balloon 1 only. Since the displacement prevention mechanism 8 is fixed onto the inner wall of the balloon 1 only and not onto the outer wall of the inner tube 3, neither the flexibility nor the rigidity is adversely affected. Accordingly, guidewire operation during the insertion of the stent to the narrowed area is no more complicated than inserting a typical balloon catheter into a narrowed area. Thus, superior maneuverability can be achieved.

The displacement prevention mechanism 8 is preferably tubular since a tubular member can be easily prepared by a known extrusion molding technique at low cost. As described in the later sections, tubular members are preferred as the displacement prevention mechanisms since tubular members can be easily subjected to various types of working (expansion, nosing, and the like). Moreover, the thickness of tubular members can be easily controlled during the extrusion molding. Thus, the rigidity at portions that carry displacement prevention mechanisms can be easily optimized, thereby achieving continuous rigidity distribution in the balloon. As a result, a stent delivery catheter having a low risk of kinking and the like that would occur at the borders between the stent 11 and the displacement prevention mechanisms 8 during the delivery of the stent 11 to a narrowed area of the body can be easily obtained. The collars disclosed in the Japanese Unexamined Patent Application Publication No. 11-128366 clearly have disadvantages regarding time and manufacturing cost since optimizing the rigidity by changing thickness, i.e., shape, of the collars is problematic due to the shape of the collars.

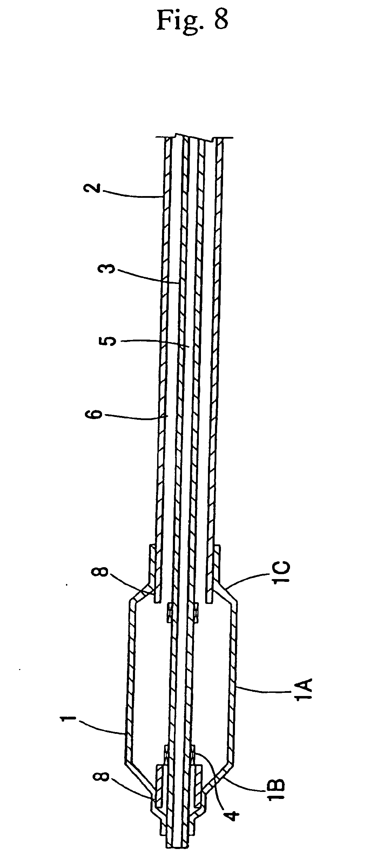

As described above, the displacement prevention mechanisms 8 are preferably disposed at the distal end and the proximal end of the balloon 1. However, instead of the embodiment having tubular members at the distal end and the proximal end of the balloon 1 (Fig. 7), the outer tube 2 may be extended so that the portion of the outer tube inside the balloon 1 can function as the displacement prevention mechanism 8 (Fig. 8). When the outer tube 2 extends into the balloon 1, the step of fixing the proximal-end displacement prevention mechanism 8 onto the balloon 1 can be omitted. Thus, the manufacturing process can be streamlined.

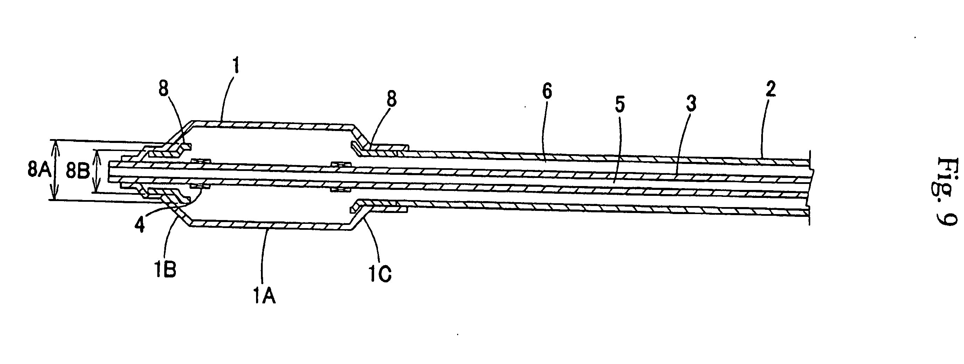

In order to further efficiently prevent displacement of the stent 11, it is preferable to provide the displacement prevention mechanisms 8 at the tapered segments 1B and 1C only and not at the straight tubular segment 1A. The outer diameter D2 of the portion of the displacement prevention mechanism 8 extending into the balloon 1 and the outer diameter D1 of the portion of the displacement prevention mechanism 8 affixed onto the balloon 1 preferably satisfy the relationship 1 ≤ (D2/D1) (Figs. 9 to 11).

According to this structure, when the balloon 1 is folded, the outer diameter D2 of the displacement prevention mechanisms 8 inside the tapered segments 1B and 1C becomes larger than the outer diameter D1 of the displacement prevention mechanism 8 at the portion affixed to the balloon 1. Accordingly, the outer diameter of each of the tapered segments 1B and 1C of the collapsed balloon can be efficiently made larger than the outer diameter of the undeployed stent 11, thereby preventing the displacement of the stent 11 on the surface of the balloon 1 and falling off of the stent 11 from the catheter.

When (D2/D1) > 2, the outer diameter of the tapered segments 1B and 1C of the collapsed balloon 1 becomes significantly larger than that of the undeployed stent 11. Accordingly, the outer diameter of the collapsed tapered segments 1B and 1C may cause friction and may often obstruct placing the stent 11 at a desired narrowed area. Thus, more preferably, the relationship 1 ≤ (D2/D1) ≤ 2 is satisfied.

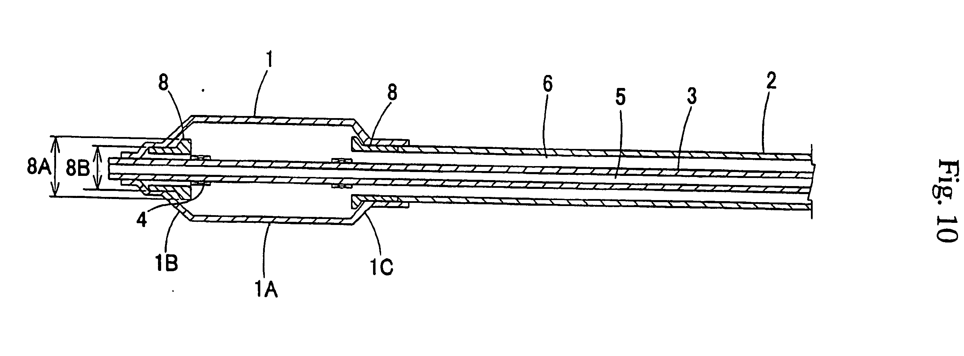

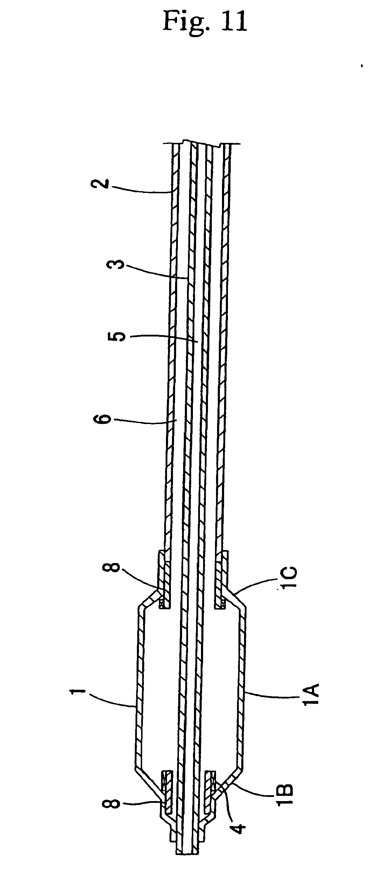

Regarding the displacement prevention mechanisms 8, any method may be employed to differentiate the outer diameter D1 at the affixed portion from the outer diameter D2 of the portion extending into the balloon 1. Examples of the method include the following: one end of a tubular member having the outer diameter D1 may be expanded to have the outer diameter D2; one end of a tubular member having the outer diameter D2 may be narrowed to have the outer diameter D1 (Fig. 9); one end of a tubular member having the outer diameter D1 may be subjected to wall thickening so as to increase the outer diameter to D2 (Fig. 10); and one end of a first tubular member having the outer diameter D1 may be joined with a second tubular member to increase the outer diameter to D2 (Fig. 11). Here, according to the method shown in Fig. 11, the second tubular member joined with the end of the first tubular member having the outer diameter D1 is preferably a tubular, ring-shaped, or letter-C shaped member that can function as a radiopaque marker 4.

By affixing the displacement prevention mechanisms 8 to the interior of the balloon 1, the area of the cross-section of the inflation lumen 6 may be decreased at the proximal-end portion of the balloon 1 and delay in in-/deflation time of the balloon may occur as a result. On the other hand, in order to secure a large enough area of the cross-section of the inflation lumen 6, the outer diameter of the affixed portion becomes large due to the affixation of the displacement prevention mechanism 8. As a result, friction may occur during insertion of the catheter. Accordingly, the displacement prevention mechanisms 8 are preferably composed of a material that can melt-bond with the balloon 1 to reduce the outer diameter of the affixed portion.

The materials of the displacement prevention mechanism 8 and the balloon 1 may be any as long as they can be melt-bonded with each other. For example, any combination selected from polyolefins, polyolefin elastomers, polyesters, polyester elastomers, polyamides, polyamide elastomers, polyurethane, polyurethane elastomers, and the like may be employed as long as the selected materials are fusible with each other. A blend material containing two or more of these resin materials or a multilayer-structure material having layers of two or more of these resin materials may also be used as long as the materials can be melt-bonded with each other. In view of general properties (such as pressure resistance required to sufficiently deploy the stent, flexibility that can track tortuous areas, recrossability, i.e., narrowed area reentry ability, compliance characteristics, and the like) required for stent-deploying balloons, the combination of a balloon 1 composed of a polyamide elastomer or a blend containing polyamide elastomers and displacement prevention mechanisms 8 composed of a polyamide or a polyamide elastomer is preferred. The combination of a balloon 1 composed of a polyester elastomer or a blend containing polyester elastomers and a displacement prevention mechanism 8 composed of polyester or a polyester elastomer is also preferred.

The method of joining the balloon 1 to the outer tube 2 and the inner tube 3 is not particularly limited. Any known technique, i.e., bonding with an adhesive, bonding by fusion, or the like, can be employed. The composition, the chemical structure, and the cure method of the adhesive used are not particularly limited. Regarding the composition and the chemical structure, an adhesive of a urethane type, a silicone type, an epoxy type, a cyanoacrylate type, or the like may be employed. Regarding the cure method, an adhesive of a two-liquid mix type, a UV-curable type, a moisture absorption curing type, a heat curing type, a radiation curing type, or the like may be employed. It is preferable to use an adhesive that can yield a rigidity at the joined portions such that the rigidity changes continuously over the balloon 1 and the outer tube 2 and over the balloon 1 and the inner tube 3. The adhesive may be selected while taking into account the rigidity of the balloon 1, the outer tube 2, and the inner tube 3.

The balloon 1 can be prepared by a suitable method, such as dip molding, blow molding, or the like. Blow molding is preferred to achieve a pressure resistance sufficient for expanding stent-deploying balloons. In particular, first, a tubular parison having a predetermined size is formed by extrusion molding. The tubular parison is then placed in a die having a shape corresponding to the shape of the balloon and is biaxially stretched in the axis direction and the diameter direction to form a balloon having the same shape as the die. The biaxial stretching may be performed with heating or may be performed more than once. The stretching in the axis direction may be performed at the same time with, before, or after the stretching in the diameter direction. Annealing may be performed to stabilize the shape and the dimensions of the balloon.

The material of the inner tube 3 in no way affects the effect of the present invention and may be any. When the coaxial structure shown in Fig. 3 is employed, the inner tube 3 may be composed of polyolefin, a polyolefin elastomer, polyester, a polyester elastomer, polyamide, a polyamide elastomer, polyurethane, a polyurethane elastomer, or the like. However, since the guidewire lumen 5 is defined by the inner wall of the inner tube 3, polyethylene, in particular, high-density polyethylene, is preferred in view of slidability of the guidewire. Moreover, the inner tube 3 may have a multilayer structure in which the innermost layer for securing slidability of the guidewire is composed of high-density polyethylene and the outermost layer is composed of a material adhesive or fusible with the balloon 1. In order to further enhance the slidability of the guidewire, the inner wall of the inner tube 3 may be provided with lubricating coating of silicon, polytetrafluoroethylene, or the like. The same material selection may be made in other structures such as the biaxial structure.

As with the inner tube 3, the material of the outer tube 2 is not particularly limited. Polyolefin, a polyolefin elastomer, polyester, a polyester elastomer, polyamide, a polyamide elastomer, polyurethane, a polyurethane elastomer, or the like may be used.

Preferable examples of the material of a hub 9 include polycarbonates, polyamides, polyurethanes, polysulfones, polyarylates, styrene-butadiene copolymers, and polyolefins.

Moreover, it is possible to mount the radiopaque marker 4 at the end of the displacement prevention mechanism 8 inside the balloon 1 (Fig. 11). In this manner, the radiopaque marker 4 usually provided on the outer wall of the inner tube 3 is no longer necessary. Accordingly, discontinuous change in rigidity of the inner tube 3 resulting from affixation of the radiopaque marker 4 and kinking of inner tube 3 during insertion of the catheter resulting from the discontinuity in rigidity can be eliminated. As a result, high maneuverability is achieved.

The material of the radiopaque marker 4 may be any as long as it does not transmit X-rays. The material may be of any type, i.e., metal or resin. Moreover, the method of affixing the radiopaque marker 4 is not particularly limited.

The outer wall of the stent delivery catheter may be provided with hydrophilic coating to facilitate insertion. In particular, it is preferable to provide hydrophilic coating to an area that comes into contact with blood. Here, the hydrophilic coating is preferably of a type that exhibits lubricity when the coating comes into contact with blood. The type of the hydrophilic coating is not particularly limited. Preferable examples thereof include hydrophilic polymers such as poly(2-hydroxyethylmethacrylate), polyacrylamide, and polyvinylpyrrolidone or any combinations of these. The coating method is also not limited.

Hydrophobic coating may be provided on the outer wall of the balloon 1 in order to facilitate positioning by preventing slipping of the balloon 1 during post-dilation after the stent deployment. The type of hydrophobic coating may be any but is preferably of a hydrophobic polymer such as silicon. The coating method is also not limited. The stent 11 may be composed of any material as long as it is of a balloon-expandable type. A preferable example of the material is stainless steel such as SUS316. The design and the like of the stent 11 are not particularly limited.

Specific examples of the invention and comparative examples will now be discussed below in detail. Note that the scope of the present invention is not limited by the examples described below.

### (EXAMPLE 1)

A tubular parison (inner diameter: 0.51 mm, outer diameter: 1.02 mm) was prepared by extrusion molding using a polyamide elastomer (trade name: PEBAX7033SA01, manufactured by Elf Atochem). The parison was subjected to a biaxial stretching blow molding to form a balloon having an outer diameter of 3.0 mm at a straight tubular segment and an inner diameter of 0.95 mm at portions to be jointed with displacement prevention mechanisms.

An inner tube (inner diameter: 0.42 mm, outer diameter: 0.56 mm) and an outer tube (inner diameter: 0.71 mm, outer diameter: 0.88 mm) were prepared by extrusion molding using a polyamide elastomer (trade name: PEBAX7233SA01, manufactured by Elf Atochem).

A displacement prevention mechanism at the distal end of the balloon was a tubular member (inner diameter: 0.65 mm, outer diameter: 0.79 mm) prepared by extrusion molding using a polyamide elastomer (trade name: PEBAX7033SA01, manufactured by Elf Atochem).

A displacement prevention mechanism at the proximal end of the balloon was a tubular member (inner diameter: 0.71 mm, outer diameter: 0.88 mm) prepared by extrusion molding using a polyamide elastomer (trade name: PEBAX7033SA01, manufactured by Elf Atochem).

The stent was prepared by electropolishing a SUS316L tube (inner diameter: 1.80 mm, outer diameter: 2.05 mm) cut to have a predetermined pattern by laser processing.

The distal-end displacement prevention mechanism and the proximal-end displacement prevention mechanism were melt-bonded with the balloon. Subsequently, the outer tube was melt-bonded with the balloon. A notch was formed in the outer tube at a position 200 mm distant from the joint between the outer tube and the balloon in the direction of the proximal end. The notch was formed by cutting the outer wall of the outer tube by approximately 180° in the circumferential direction. The inner tube was inserted through the notch so as to coaxially position the inner tube and the outer tube to form a double tube. One end of the inner tube was coincident with the notch and the other end of the inner tube was extended across the balloon interior so that the other end protrudes from the balloon. Subsequently, the outer tube was melt-bonded with the inner tube at the notch, and the balloon was melt-bonded to the inner tube at the distal end of the balloon. During the bonding, a core of a predetermined size coated with a high-lubricity material such as polytetrafluoroethylene was used as required in order to maintain the inflation lumen or the guidewire lumen.

The balloon was collapsed to a tri-set shape (the number of folded wings being three) by reducing the pressure inside the balloon. The undeployed stent was mounted on the straight tubular segment of the balloon, and a heat-shrinkable tube mounted on the outer wall of the stent was shrunk to put the stent into close contact with the outer wall of the balloon. Finally, the heat-shrinkable tube was removed to prepare a distal-end sample of a rapid-exchange stent delivery catheter.

### (EXAMPLE 2)

A sample was prepared as in EXAMPLE 1 except that the outer tube was used as the displacement prevention mechanism at the proximal end of the balloon.

### (EXAMPLE 3)

A sample was prepared as in EXAMPLE 1 with the exception of the following. The same tubular member as in EXAMPLE 1 was used as the displacement prevention mechanism at the distal end of the balloon. The outer diameter of the tubular member was increased to 1.20 mm, and the portion with the increased outer diameter was extended into the balloon tapered segment at the distal end of the balloon. The same tubular member as in EXAMPLE 1 was used as the displacement prevention mechanism at the proximal end of the balloon. The outer diameter of one end of the tubular member was increased to 1.20 mm, and the portion with the increased outer diameter was extended into the balloon tapered segment at the proximal end. Moreover, the inner diameter of the portions of the balloon jointed with the displacement prevention mechanisms was adjusted to 1.25 mm.

### (EXAMPLE 4)

A sample was prepared as in EXAMPLE 1 with the exception of the following. A radiopaque marker (inner diameter: 0.83 mm, outer diameter: 0.90 mm, composed of platinum) was affixed to the end of the displacement prevention mechanism at the balloon distal end using a two-liquid mix urethane adhesive (UR0531, manufactured by H. B. Fuller Company) so that the radiopaque marker was located at the border between the straight tubular segment and the tapered segment at the distal end of the balloon. Another radiopaque marker (inner diameter: 0.92 mm, outer diameter: 1.00 mm, composed of platinum) was affixed to the end of the displacement prevention mechanism at the balloon proximal end using a two-liquid mix urethane adhesive (UR0531, manufactured by H. B. Fuller Company) so that the radiopaque marker was located at the border between the straight tubular segment and the tapered segment at the proximal end of the balloon. Moreover, the inner diameter of the portions of the balloon jointed with the displacement prevention mechanism was adjusted to 1.25 mm.

### (COMPARATIVE EXAMPLE 1)

A sample was prepared as in EXAMPLE 1 but without the displacement prevention mechanisms at the distal end and the proximal end of the balloon.

### (EXAMPLE 5)

A sample was prepared as in EXAMPLE 1 except for the following. The same tubular member (first tubular member) as in EXAMPLE 1 was used to form each of the displacement prevention mechanisms at the distal and proximal ends of the balloon. Another tubular member (second tubular member, inner diameter: 0.95 mm, outer diameter: 2.00 mm) prepared by extrusion molding using a polyamide elastomer (trade name: PEBAX7033SA01, manufactured by Elf Atochem) was joined to one end of each first tubular member by melt bonding. The jointed portions were placed in tapered segments at the distal and proximal ends of the balloon, respectively. The inner diameter of the jointed portions of the balloon jointed with the displacement prevention mechanisms were adjusted to 2.05 mm.

### (EXAMPLE 6)

A tubular parison (inner diameter: 0.43 mm, outer diameter: 0.89 mm) was prepared by extrusion molding using a polyester elastomer (trade name: Pelprene S-6001, manufactured by Toyobo Co., Ltd.). The parison was formed into a balloon by biaxial stretching blow molding having a an outer diameter of 3.0 mm at the straight tubular segment and an inner diameter of 0.95 mm at the portions jointed to displacement prevention mechanisms. An inner tube (inner diameter: 0.42 mm, outer diameter: 0.56 mm) was prepared by extrusion molding using high-density polyethylene (trade name: HY540, manufactured by Mitsubishi Chemical Corporation) and an outer tube (inner diameter: 0.71 mm, outer diameter: 0.88 mm) was prepared by extrusion molding using a polyester elastomer (trade name: Pelprene S-6001, Toyobo Co., Ltd.).

The displacement prevention mechanism at the distal end of the balloon was a tubular member (inner diameter: 0.65 mm, outer diameter: 0.79 mm) prepared by extrusion molding using a polyester elastomer (trade name: Pelprene S-3001, manufactured by Toyobo Co., Ltd.). The displacement prevention mechanism at the proximal end of the balloon was a tubular member (inner diameter: 0.71 mm, outer diameter: 0.88 mm) prepared by extrusion molding using a polyester elastomer (trade name: Pelprene S-3001, manufactured by Toyobo Co., Ltd.).

The stent was prepared by electropolishing a SUS316L tube (inner diameter: 1.80 mm, outer diameter: 2.05 mm) cut to have a predetermined pattern by laser processing.

The displacement prevention mechanisms at the distal and proximal ends were melt-bonded with the balloon, and the outer tube was melt-bonded with the balloon. A notch was formed in the outer tube at a position 200 mm distant from the joint between the outer tube and the balloon in the proximal-end direction. Here, the notch was formed by cutting the outer wall of the outer tube by approximately 180° in the circumferential direction. The inner tube was inserted through the notch so as to coaxially position the inner tube and the outer tube to form a double tube. One end of the inner tube was coincident with the notch and the other end of the inner tube was extended across the balloon interior so that the other end protrudes from the balloon. Subsequently, the outer tube was bonded to the inner tube at the notch using a two-liquid mix urethane adhesive (UR0531, manufactured by H.B. Fuller Company). The balloon was bonded to the inner tube at the distal end of the balloon by the same adhesive. During the bonding, a core having a predetermined size coated with a high-lubricity material such as polytetrafluoroethylene was used as required in order to support the inflation lumen. Prior to the bonding, the inner tube was subjected to oxygen plasma treatment.

The balloon was collapsed to a tri-set shape (the number of folded wings being three) by reducing the pressure inside the balloon. The undeployed stent was mounted on the straight tubular segment of the balloon, and a heat-shrinkable tube mounted on the outer wall of the stent was shrunk to put the stent into close contact with the outer wall of the balloon. Finally, the heat-shrinkable tube was removed to prepare a distal-end sample of a rapid-exchange stent delivery catheter.

### (EXAMPLE 7)

A sample was prepared as in EXAMPLE 6 except that the outer tube was used as the displacement prevention mechanism at the proximal end of the balloon.

### (EXAMPLE 8)

A sample was prepared as in EXAMPLE 6 with the exception of the following. The same tubular member as in EXAMPLE 5 was used as the displacement prevention mechanism at the distal end of the balloon. The outer diameter of the tubular member was increased to 1.20 mm, and the portion with the increased outer diameter was placed in the balloon tapered segment at the distal end of the balloon. The same tubular member as in EXAMPLE 5 was used as the displacement prevention mechanism at the proximal end of the balloon. The outer diameter of one end of the tubular member was increased to 1.20 mm, and the portion with the increased outer diameter was placed inside the balloon tapered segment at the proximal end. Moreover, the inner diameter of the portions of the balloon joined with the displacement prevention mechanisms was adjusted to 1.25 mm.

### (EXAMPLE 9)

A sample was prepared as in EXAMPLE 6 with the exception of the following. A radiopaque marker (inner diameter: 0.83 mm, outer diameter: 0.90 mm, composed of platinum) was affixed to the end of the displacement prevention mechanism at the balloon distal end using a two-liquid mix urethane adhesive (UR0531, manufactured by H. B. Fuller Company) so that the radiopaque marker was located at the border between the straight tubular segment and the tapered segment at the distal end of the balloon. Another radiopaque marker (inner diameter: 0.92 mm, outer diameter: 1.00 mm, composed of platinum) was affixed to the end of the displacement prevention mechanism at the balloon proximal end using a two-liquid mix urethane adhesive (UR0531, manufactured by H. B. Fuller Company) so that the radiopaque marker was located at the border between the straight tubular segment and the tapered segment at the proximal end of the balloon. Moreover, the inner diameter of the portions of the balloon joined with the displacement prevention mechanisms was adjusted to 1.25 mm.

### (COMPARATIVE EXAMPLE 2)

A sample was prepared as in EXAMPLE 6 but without the displacement prevention mechanisms at the distal end and the proximal end of the balloon.

### (EXAMPLE 10)

A sample was prepared as in EXAMPLE 6 except for the following. The same tubular member (first tubular member) as in EXAMPLE 5 was used to form each of the displacement prevention mechanisms at the distal and proximal ends of the balloon. Another tubular member (second tubular member, inner diameter: 0.95 mm, outer diameter: 2.00 mm) prepared by extrusion molding using a polyester elastomer (trade name: Pelprene S-3001, manufactured by Toyobo Co., Ltd.) was joined to one end of each first tubular member by melt bonding. The jointed portions were placed inside the tapered segments at the distal and proximal ends of the balloon. The inner diameter of the portions of the balloon jointed with the displacement prevention mechanisms were adjusted to 2.05 mm.

### (Assessment of stent displacement prevention properties according to the invention)

Each sample was horizontally moved while holding the portion carrying the stent to qualitatively determine whether displacement or falling of the stent occurs readily.

### (Assessment of the insertion characteristics to the body according to the invention)

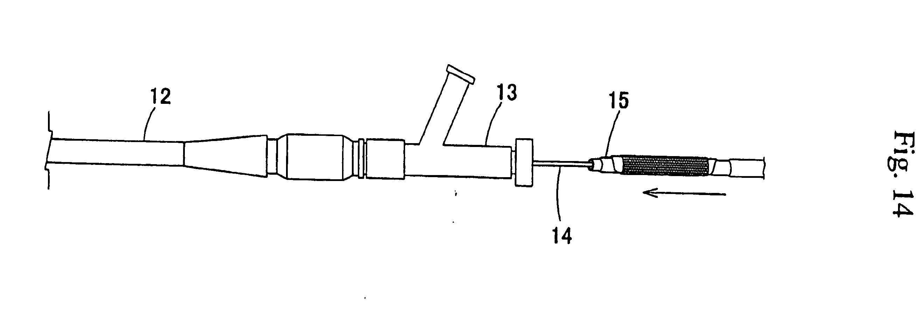

The insertion maneuverability of the sample into a body was assessed. The assessment was carried out in a system including a guide catheter 12, a hemostatic valve 13, and a guidewire 14 arranged as shown in Fig. 14, in which water is circulated inside the guide catheter 12 and the hemostatic valve 13. The sample was inserted from the inlet of the hemostatic valve 13 to evaluate the insertion maneuverability. In the assessment, Zuma II (7 Fr, JL 4.0, manufactured by Medtronic AVE Corporation) was used as the guide catheter 12, and BMW (0.014", manufactured by Guidant Corporation) was used as the guidewire 14.

**Table 1 Assessment Results of the Invention**

| | Displacement properties | Insertion characteristics | Displacement prevention mechanism at balloon distal end | | | Displacement prevention mechanism at balloon proximal end | | |
|---|---|---|---|---|---|---|---|---|
| | | | D1 (mm) | D2 (mm) | D2/D1 | D1 (mm) | D2 (mm) | D2/D1 |
| EXAMPLE 1 | ○ | ○ | 0.79 | 0.79 | 1.00 | 0.88 | 0.88 | 1.00 |
| EXAMPLE 2 | ○ | ○ | 0.79 | 0.79 | 1.00 | 0.88 | 0.88 | 1.00 |
| EXAMPLE 3 | ○ | ○ | 0.79 | 1.20 | 1.52 | 0.88 | 1.20 | 1.36 |
| EXAMPLE 4 | ○ | ○ | 0.79 | 0.90 | 1.14 | 0.88 | 1.00 | 1.14 |
| EXAMPLE 5 | ○ | Δ | 0.79 | 2.00 | 2.53 | 0.88 | 2.00 | 2.27 |
| COMPARATIVE EXAMPLE 1 | × | ○ | - | - | - | - | - | - |
| EXAMPLE 6 | ○ | ○ | 0.79 | 0.79 | 1.00 | 0.88 | 0.88 | 1.00 |
| EXAMPLE 7 | ○ | ○ | 0.79 | 0.79 | 1.00 | 0.88 | 0.88 | 1.00 |
| EXAMPLE 8 | ○ | ○ | 0.79 | 1.20 | 1.52 | 0.88 | 1.20 | 1.36 |
| EXAMPLE 9 | ○ | ○ | 0.79 | 0.90 | 1.14 | 0.88 | 1.00 | 1.14 |
| EXAMPLE 10 | ○ | Δ | 0.79 | 2.00 | 2.53 | 0.88 | 1.00 | 2.27 |
| COMPARATIVE EXAMPLE 2 | × | ○ | - | - | - | - | - | - |

| | | | | | | | | |
|---|---|---|---|---|---|---|---|---|
| Displacement prevention properties ○: Neither displacement nor falling of the stent occurred ×: Stent moved easily Insertion characteristics ○: Easily inserted Δ: Friction inside the guide catheter was experienced during the insertion, poor insertion maneuverability | | | | | | | | |

In EXAMPLES 1 to 10 of the invention, the stent did not move or fall off. Thus, the effect of the invention was achieved. COMPARATIVE EXAMPLES 1 and 2 frequently experienced displacement or falling-off of the stent and were thus obviously unsuitable for use in stent delivery catheters. Moreover, in EXAMPLES 5 and 10, the collapsed balloons had a slightly larger outer diameter at the tapered segments. This resulted in friction inside the guide catheter during insertion operation into the body and in poor maneuverability; however, the displacement and falling of the stent were effectively prevented.

Various examples of the stent delivery catheter according to a second aspect of the catheter, useful to understand the invention will now be described in detail with reference to the drawings. As with the first invention, the stent delivery catheter of the second invention may also be of the over-the-wire type shown in Fig. 1 or the rapid exchange type shown in Fig. 2.

In the stent delivery catheter of the second aspect, the basic structure of the catheter, the method for making the balloon 1, materials of the outer tube 2, the inner tube 3, and the hub 9, the method for joining the balloon 1 with the outer tube 2 and the inner tube 3, and the like are identical to those of the described invention. Thus, the description regarding these features is omitted, and only the features different from the described invention will be described below in detail.

The undeployed stent 11 is mounted on the collapsed balloon 1 that satisfies both the relationship (T1/T2) < 1.3, wherein T1 represents the thickness of a near-center portion of the tapered segment 1B at the distal end and T2 represents the thickness of a near-center portion of the straight tubular segment 1A, and the relationship (T3/T2) < 1.3, wherein T3 represents the thickness of a near-center portion of the tapered segment 1C at the proximal end and T2 represents the thickness of the near-center portion of the straight tubular segment 1A. In such a case, the outer diameter of the undeployed stent 11 becomes larger than the outer diameters of the straight tubular segment 1A, the tapered segment 1B at the distal end, and the tapered segment 1C at the proximal end of the balloon 1. Accordingly, friction may occur between the stent 11 and the hemostatic valve 13 or the guide catheter 12, and the stent 11 may move on the balloon or may even fall off from the catheter (the same configuration as shown in Fig. 12). Here, the term "near-center portion" means "approximately central portion" and defines the area within the range ±1 mm from the true center.

When both the relationships 1.3 s (T1/T2) and 1.3 ≤ (T3/T2) are satisfied, the collapsed distal-end tapered segment 1B and the proximal-end tapered segment 1C become bulky due to the increased thickness of the distal-end tapered segment 1B and the proximal-end tapered segment 1C. Thus, the outer diameters thereof become larger than the outer diameter of the undeployed stent 11. As a result, the stent 11 can be prevented from moving on the surface of the balloon 1 or falling from the catheter (the same appearance as in Fig. 13).

When the stent 11 is used as a coronary artery stent, the thickness of the metal components constituting the stent is preferably 100 µm to 150 µm. Taking this into consideration, the relationships 1.6 ≤ (T1/T2) and 1.6 ≤ (T3/T2) are preferably satisfied in order to effectively prevent the stent 11 from moving or falling.

When the relationships 2.5 < (T1/T2) and 2.5 < (T3/T2) are satisfied, the collapsed distal-end tapered segment 1B and proximal-end tapered segment 1C become bulky due to the thickness of the distal-end tapered segment 1B and proximal-end tapered segment 1C as described above. However, the collapsed distal-end tapered segment 1B and proximal-end tapered segment 1C become excessively bulky in this case and thus have an excessively larger outer diameter than the undeployed stent 11. According to this arrangement, during the insertion of the stent delivery catheter to a narrowed area along a tortuous blood vessel or a guide catheter, the trackability at the tortuous areas is dramatically degraded and maneuvering becomes difficult. Moreover, since the collapsed distal-end tapered segment 1B and proximal-end tapered segment 1C are excessively bulky, the inner wall of the blood vessel may be damaged. Furthermore, in extracting the balloon catheter from inside the body after the deployment and deflation of the stent 11, the balloon 1 may be caught inside the stent 11, thereby disrupting the extraction operation.

In view of the above, the thickness T1 of the near-center portion of the distal-end tapered segment 1B and the thickness T2 of the near-center portion of the straight tubular segment 1A preferably satisfy the relationship 1.3 s (T1/T2) ≤ 2.5; at the same time, the thickness T3 of the near-center portion of the proximal-end tapered segment 1C and the thickness T2 of the near-center portion of the straight tubular segment 1A preferably satisfy the relationship 1.3 ≤ (T3/T2) ≤ 2.5. More preferably, the thickness T1 of the near-center portion of the distal-end tapered segment 1B and the thickness T2 of the near-center portion of the straight tubular segment 1A satisfy the relationship 1.6 ≤ (T1/T2) ≤ 2.5; at the same time, the thickness T3 of the near-center portion of the proximal-end tapered segment 1C and the thickness T2 of the near-center portion of the straight tubular segment 1A satisfy the relationship 1.6 s (T3/T2) ≤ 2.5.

It should be noted here that although Fig. 13 shows the invention in which the presence of the displacement prevention mechanisms 8 is apparent from the outline of the collapsed balloon 1, the second aspect also has the same appearance at the distal end of the catheter since the collapsed distal-end tapered segment 1B and tapered segment 1C are bulkier than the straight tubular segment 1A when the balloon 1 is collapsed by folding. In other words, since the thickness balloon 1 of the near-center portion of the distal-end tapered segment 1B, the thickness T2 of the near-center portion of the straight tubular segment 1A, and the thickness T3 of the near-center portion of the tapered segment 1C of the balloon 1 are adjusted as such, the collapsed distal-end tapered segment 1B and proximal-end tapered segment 1C prevent the stent 11 from moving, thereby functioning as the displacement prevention mechanisms 8 of the invention.

It is obvious to persons skilled in the art that the thicknesses T1 and T3 can be varied without changing T2 by adjusting the amount of stretching in the axial direction or by adjusting the balance of the biaxial stretching. In order to more effectively change T1 and T3 only, it is preferable to perform biaxial stretching two or more times under heating. More preferably, a device that can heat each of the straight tubular segment 1A, the distal-end tapered segment 1B, and the proximal-end tapered segment 1C independently is used to selectively perform or not perform biaxial stretching at predetermined areas. Moreover, the stretching in the axis direction may be performed at the same time with, before, or after the stretching in the diameter direction. Annealing may be performed to stabilize the shape and the dimension of the balloon. As is apparent from the above, the displacement of the stent 11 can be prevented by optimizing the conditions of the biaxial stretching without complicating the manufacturing process.

The material of the balloon 1 may be any material that can be subjected to biaxial stretching. Such a material does not adversely affect the advantages of the present invention. Examples of the material include polyolefins, polyolefin elastomers, polyesters, polyester elastomers, polyamides, polyamide elastomers, polyurethanes, and polyurethane elastomers. Polyesters, polyester elastomers, polyamides, and polyamide elastomers are particularly preferred in view of achieving pressure resistance that can sufficiently deploy the stent 11.

The second aspect will now be described by way of EXAMPLES and COMPARATIVE EXAMPLES. Naturally, the invention is in no way limited by the examples below.

### (EXAMPLE 11)

A tubular parison (inner diameter: 0.51 mm, outer diameter: 1.02 mm) was prepared by extrusion molding using a polyamide elastomer (trade name: PEBAX7033SA01, manufactured by Elf Atochem). The parison was subjected to a biaxial stretching blow molding to form a balloon. This balloon had an outer diameter of 3.00 mm at the straight tubular segment, a thickness of 26 µm at the near-center portion of the distal-end tapered segment, a thickness of 19 µm at the near-center portion of the straight tubular segment, and a thickness of 25 µm at the near-center portion of the proximal-end tapered segment. The thickness of the near-center portions of the distal-end tapered segment, the tubular segment, and the proximal-end tapered segment was measured with a laser confocal displacement meter (Controller: LT-8100, Head: LT-8010, Camera Unit: LT-V201, all manufactured by Keyence Corporation).

An inner tube (inner diameter: 0.42 mm, outer diameter: 0.56 mm) and an outer tube (inner diameter: 0.71 mm, outer diameter: 0.88 mm) were prepared by extrusion molding using a polyamide elastomer (trade name: PEBAX7233SA01, manufactured by Elf Atochem).

A stent was prepared by electropolishing a SUS316L tube (inner diameter: 1.80 mm, outer diameter: 2.05 mm) cut to have a predetermined pattern by laser processing.

After the outer tube was joined with the balloon by fusion bonding, a notch was formed in the outer tube at a position 200 mm distant from the joint between the outer tube and the balloon in the direction of the proximal end. The notch was formed by cutting the outer wall of the outer tube by approximately 180° in the circumferential direction. The inner tube was inserted through the notch so as to coaxially position the inner tube and the outer tube to form a double tube. One end of the inner tube was coincident with the notch and the other end of the inner tube was extended across the balloon interior so that the other end protruded from the balloon. Subsequently, the outer tube was joined with the inner tube at the notch by melt bonding, and the balloon was joined to the inner tube at the distal end of the balloon by melt bonding. During the bonding, a core having a predetermined size coated with a high-lubricity material such as molding polytetrafluoroethylene was used as required in order to support the inflation lumen or the guidewire lumen.

The balloon was collapsed to a tri-set shape (the number of folded wings being three) by reducing the pressure inside the balloon. The undeployed stent was mounted on the straight tubular segment of the balloon, and a heat-shrinkable tube mounted on the outer wall of the stent was shrunk to put the stent into close contact with the outer wall of the balloon. Finally, the heat-shrinkable tube was removed to prepare a distal-end sample of a rapid-exchange stent delivery catheter.

### (EXAMPLE 12)

A sample was prepared as in EXAMPLE 11 except that the conditions for biaxial stretching blow molding were changed so that the outer diameter of the straight tubular segment was 2.95 mm, the thickness of the near-center portion of the distal-end tapered segment was 38 µm, the thickness of the near-center portion of the straight tubular segment was 21 µm, and the thickness of the near-center portion of the proximal-end tapered segment was 40 µm.

### (EXAMPLE 13)

A sample was prepared as in EXAMPLE 11 except that the conditions for biaxial stretching blow molding were changed so that the outer diameter of the straight tubular segment was 2.97 mm, the thickness of the near-center portion of the distal-end tapered segment was 34 µm, the thickness of the near-center portion of the straight tubular segment was 20 µm, and the thickness of the near-center portion of the proximal-end tapered segment was 33 µm.

### (EXAMPLE 14)

A sample was prepared as in EXAMPLE 11 except for the following. A tubular parison (inner diameter: 0.51 mm, outer diameter: 1.12 mm) was prepared by extrusion molding using a polyamide elastomer (trade name: PEBAX7033SA01, manufactured by Elf Atochem). The parison was subjected to a biaxially stretching blow molding to form a balloon. This balloon had an outer diameter of 2.92 mm at the straight tubular segment, a thickness of 57 µm at the near-center portion of the distal-end tapered segment, a thickness of 23 µm at the near-center portion of the straight tubular segment, and a thickness of 53 µm at the near-center portion of the proximal-end tapered segment.

### (COMPARATIVE EXAMPLE 3)

A sample was prepared as in EXAMPLE 11 except that the conditions for biaxial stretching blow molding were changed so that the outer diameter of the straight tubular segment was 3.02 mm, the thickness of the near-center portion of the distal-end tapered segment was 24 µm, the thickness of the near-center portion of the straight tubular segment was 19 µm, and the thickness of the near-center portion of the proximal-end tapered segment was 23 µm.

### (COMPARATIVE EXAMPLE 4)

A sample was prepared as in EXAMPLE 14 except that the conditions for biaxial stretching blow molding were changed so that the outer diameter of the straight tubular segment was 2.91 mm, the thickness of the near-center portion of the distal-end tapered segment was 62 µm, the thickness of the near-center portion of the straight tubular segment was 23 µm, and the thickness of the near-center portion of the proximal-end tapered segment was 60 µm.

### (Assessment of the stent displacement prevention properties)

Each sample was horizontally moved while holding the portion carrying the stent to qualitatively determine whether displacement or the falling of the stent readily occurs.

### (Assessment of the insertion characteristics)

The insertion maneuverability of each sample into a body was assessed. The assessment was carried out in a system including a guide catheter 12, a hemostatic valve 13, and a guidewire 14 arranged as shown in Fig. 14, in which water is circulated inside the guide catheter 12 and the hemostatic valve 13. The sample was inserted from the inlet of the hemostatic valve 13 to evaluate the ease of performing insertion.

**Table 2 The assessment Results of Second Aspect**

| | Displacement properties | Insertion characteristics | Thickness (µm) | | | T1/T2 | T3/ T2 |
|---|---|---|---|---|---|---|---|
| | | | Near-center portion of distal-end tapered segment T1 | Near-center portion of straight tubular segment T2 | Near-center portion of proximal-end tapered segment T3 | | |
| EXAMPLE 11 | Δ | ○ | 26 | 19 | 25 | 1.37 | 1.32 |
| EXAMPLE 12 | O | O | 38 | 21 | 40 | 1.81 | 1.90 |
| EXAMPLE 13 | O | O | 34 | 20 | 33 | 1.70 | 1.65 |
| EXAMPLE 14 | O | O | 57 | 23 | 53 | 2.48 | 2.30 |
| COMPARATIVE EXAMPLE 3 | × | O | 24 | 19 | 23 | 1.26 | 1.21 |
| COMPARATIVE EXAMPLE 4 | O | × | 62 | 23 | 60 | 2.70 | 2.61 |

| | | | | | | | |
|---|---|---|---|---|---|---|---|
| Displacement prevention properties O:Neither displacement nor falling of the stent occurred* Δ: Stent moved slightly ×: Stent moved easily Insertion characteristics O: Easily inserted Δ: Friction inside the guide catheter was experienced during the insertion, poor insertion maneuverability | | | | | | | |

In EXAMPLES 11 to 14 of the second Aspect, neither displacement nor falling of stent occurred, and the insertion characteristics to the body were satisfactory.

COMPARATIVE EXAMPLE 3 readily experienced displacement or falling of the stent and was thus obviously unsuitable for use in stent delivery catheters. COMPARATIVE EXAMPLE 4 did not experiment displacement or falling of the stent; however, a large friction was produced inside the guide catheter due to excessively large outer diameters of the distal end and proximal-end tapered segments of the folded balloon. Thus, the maneuverability was poor.

It is also effective to combine the second example with the invention to prevent the undeployed stent mounted on the outer surface of the collapsed balloon from moving in the axis direction. In particular, displacement prevention mechanisms affixed only to the inner surface of the balloon can be provided to prevent the stent from moving in the longitudinal direction of the stent delivery catheter while adjusting the thickness T1 of the near-center portion of the distal-end tapered segment and the thickness T2 of the near-center portion of the near-center portion of the straight tubular segment to satisfy the relationship 1.3 s (T1/T2) ≤ 2.5 and while adjusting the thickness T3 of the near-center portion of the proximal-end tapered segment and the thickness T2 of the near-center portion of the near-center portion of the straight tubular segment to satisfy the relationship 1.3 ≤ (T3/T2) ≤ 2.5.

Various examples to understand the invention of a balloon catheter according to a third aspect will now be described in detail with reference to the drawings.

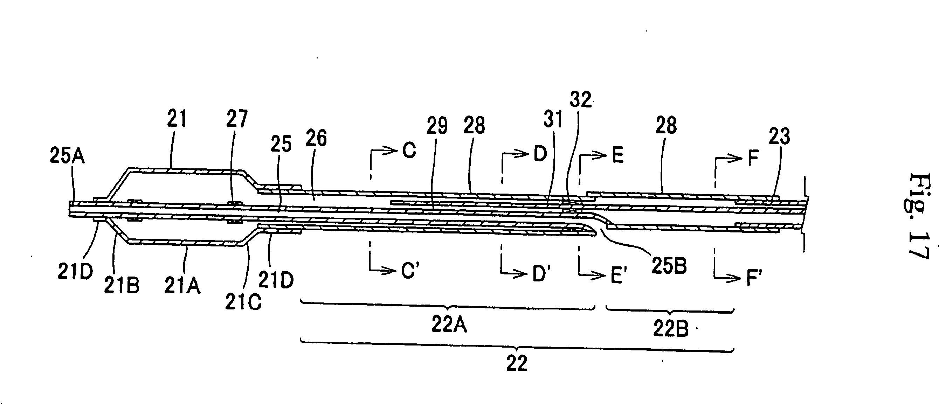

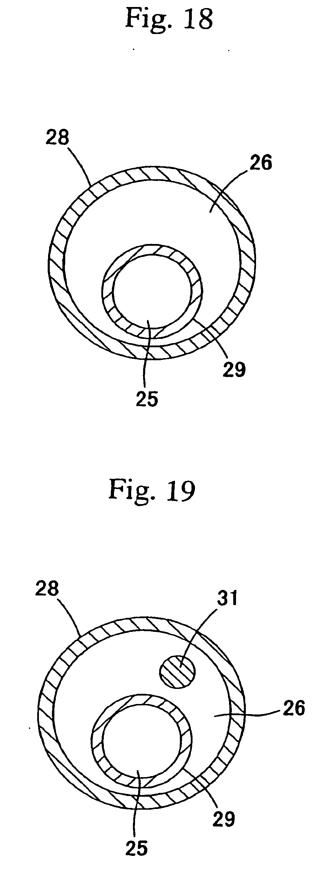

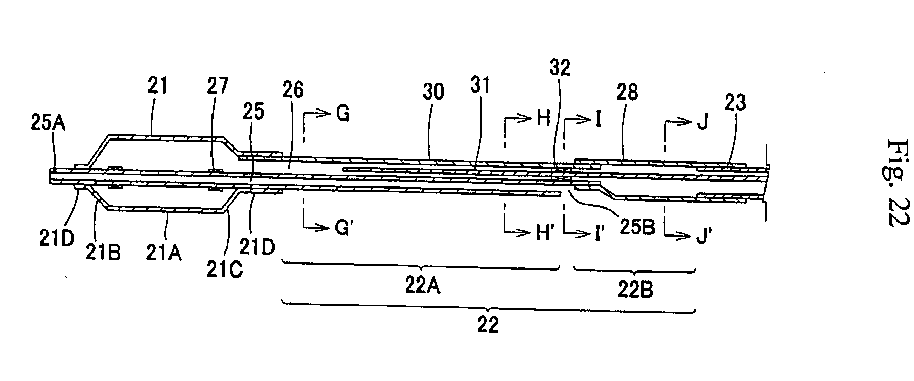

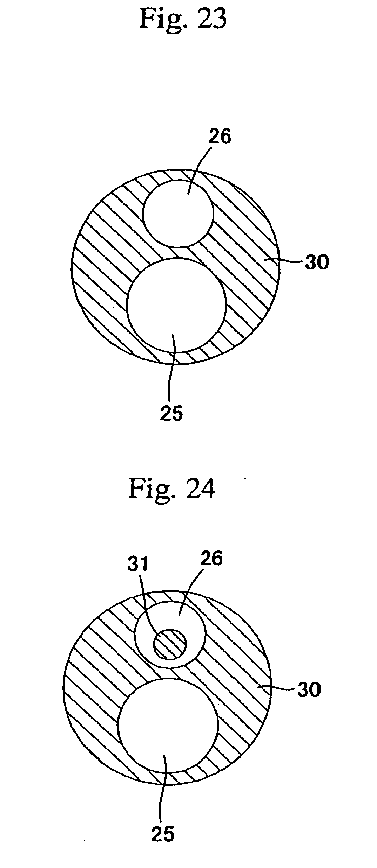

A balloon catheter suitable for use with balloon-expandable stents according to this aspect is of a rapid exchange (RX) type in which the guidewire lumen 25 is disposed only at the distal end of the balloon catheter and the proximal-end opening 25B of the guidewire lumen 25 is formed in the balloon catheter. The structure may be any as long as a distal-end segment 22A of the distal-end shaft is provided with the guidewire lumen 25 and an inflation lumen 26. In other words, as shown in Figs. 17 and 18, the distal-end segment 22A may be of a coaxial type having an outer tube 28 and an inner tube 29 coaxially provided to form a double tube; the guidewire lumen 25 defined by the inner wall of the inner tube 29; and the inflation lumen 26 defined by the inner wall of the outer tube 28 and the outer wall of the inner tube 29. Alternatively, the distal-end segment 22A may be of a biaxial type having the guidewire lumen 25 and the inflation lumen 26 arranged in parallel, as shown in Figs. 22 and 23.

The balloon catheter of this aspect is characterized in that the proximal-end opening 25B of the guidewire lumen 25 is formed in the distal-end shaft 22, that a core wire 31 for adjusting flexibility of a proximal-end segment 22B of the distal-end shaft so that the proximal-end segment 22B becomes harder than the distal-end segment 22A but softer than the proximal-end shaft 23, and that the core wire 31 is affixed on the distal-end shaft 22 only at the region near the proximal-end opening 25B of the guidewire lumen 25.

The core wire 31 is affixed to the distal-end shaft 22 only at the region near the proximal-end opening 25B of the guidewire lumen 25. Examples are shown in Figs. 17 to 21. As shown in the drawings, when the distal-end segment 22A is of the coaxial type, the core wire 31 may be affixed only at a core-wire affixation region 32 formed by filling the gap between the inner wall of the distal-end shaft, which defines the inflation lumen 26 (i.e., the gap between the inner wall of the outer tube 28 and the outer wall of the inner tube 29), and the core wire 31 with an adhesive so as to surround the core wire 31. Alternatively, the core wire 31 may be affixed only at the core-wire affixation region 32 formed by filling the gap between the inner wall of the distal-end shaft that defines the inflation lumen 26 (i.e., the gap between the inner wall of the inner tube 29 and the outer wall of the inner tube 29) and the core wire 31 with a resin so as to surround the core wire 31. However, from the standpoint of reducing the diameter of the core-wire affixation region 32 and simplifying the manufacturing process, the structure with the core-wire affixation region 32 formed by filling a molten resin is preferred, and the inner wall of the outer tube 28 and the outer wall of the inner tube 29 are preferably composed of a melt-bondable resin.

When the biaxial structure shown in Figs. 22 to 26 is employed, only the core-wire affixation region 32 formed by filling the gap between one lumen of a dual lumen tube 30, i.e., the inflation lumen 26, and the core wire 31 with an adhesive so as to surround the core wire 31 may be used for affixation. Alternatively, only the core-wire affixation region 32 formed by filling the gap with a molten resin so as to surround the core wire 31 may be used for affixation. However, for the same reason described above, a structure that uses the core-wire affixation region 32 formed by filling the gap with a molten resin is preferred.

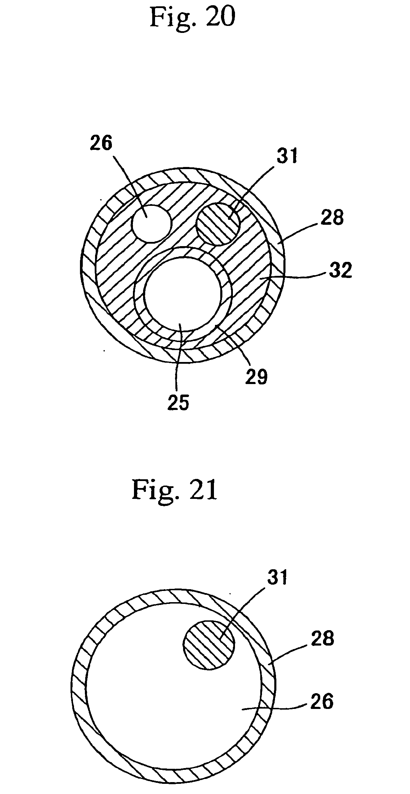

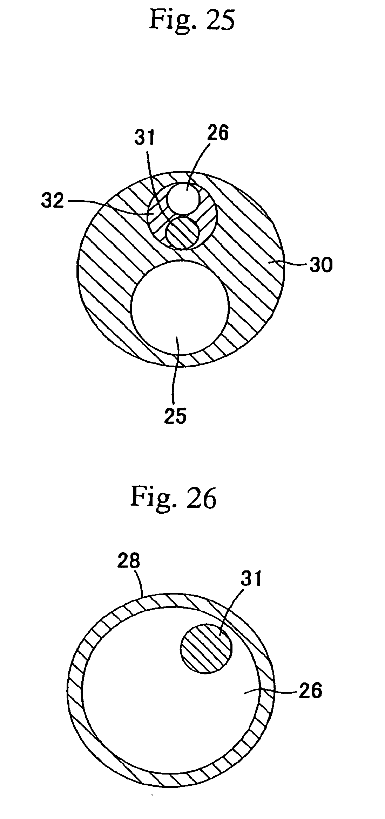

In any of the above-described methods, it becomes necessary to insert a core of predetermined shape and size to form the inflation lumen 26 at the core-wire affixation region 32. Since the core is removed after working, the outer surface of the core is preferably coated with polytetrafluoroethylene or the like to yield an inert surface. In each of Figs. 20 and 25, an example using a core having a substantially circular cross-section to form the inflation lumen 26 is shown. In these examples, the cross-section of the inflation lumen 26 is substantially circular as a result. However, the shape of the cross-section of the core does not limit the advantages of the present example. In particular, an oblong core, an elliptic core, or the like may be used while considering the workability and the required cross-sectional area of the inflation lumen 26.

When the core wire 31 is affixed to the distal-end shaft 22 at only the region near the proximal-end opening 25B of the guidewire lumen 25, the affixation of the core wire 31 can be completed at the same time with forming the proximal-end opening 25B of the guidewire lumen 25, thereby streamlining the manufacturing process. Moreover, the strength of the proximal-end opening 25B of the guidewire lumen 25 can be efficiently increased due to the adhesive or, preferably, the resin layer that surrounds the core wire 31 and fills the inflation lumen 26 adjacent to the proximal-end opening 25B of the guidewire lumen 25. Thus, it is the foremost important feature of the present aspect to affix the core wire 31 to the distal-end shaft 22 only at the region near the proximal-end opening 25B of the guidewire lumen 25.

Moreover, since the core wire 31 is not fixed in portions other than the area near the proximal-end opening 25B of the guidewire lumen 25, the step of fixing (brazing, laser-bonding, or the like) the core wire 31 and the proximal-end shaft 23 disclosed in PCT Japanese Translation Patent Publication No. 6-507105 is no longer necessary. Thus, the manufacturing process can be simplified, and the manufacturing cost can be reduced.

It is obvious to persons skilled in the art that the maneuverability of inserting the balloon catheter from outside the body along the guidewire depends upon the continuity of the rigidity in the longitudinal direction of the balloon catheter. When there is discontinuous rigidity, pushing of the balloon catheter from outside the body along the guidewire may result in kinking (breakage) of the balloon catheter. Moreover, the force applied by an operator does not efficiently transmit to the distal tip of the balloon catheter. Thus, the balloon catheter rarely passes through the stenosis lesion. From the standpoint of preventing the kinking, the core wire 31 preferably extends over the proximal-end opening 25B of the guidewire lumen 25 and resides in the distal end side, as shown in Figs. 17 and 22.

The interior of the proximal-end shaft 23 forms the inflation lumen 26. The inflation lumen 26 becomes naturally smaller as the length of the core wire 31 presiding inside the proximal-end shaft 23 increases. Thus, in order to accomplish the object of the present aspect, preferably, the core wire 31 need only reach the interior of the proximal-end shaft 23 and not the proximal end of the proximal-end shaft 23 in order to maintain good responsiveness for expansion and contraction of the balloon 21. The length of the core wire 31 extending into the interior of the proximal-end shaft 23 may be selected while considering the responsiveness for the expansion and contraction of the balloon 21, i.e., the volume inside the balloon 21 and the size of the inflation lumen 26 of each of the distal-end shaft 22 and the proximal-end shaft 23. The length of the core wire 31 is 5 to 100 mm, and preferably 10 to 50 mm. PCT Japanese Translation Patent Publication No. 9-503411 discloses a related art in which a reinforcing stylet extends from near the proximal end of the catheter shaft to the proximal side of the balloon and a preferred embodiment in which the base of the reinforcing stylet is embedded in the hub. In other words, according to this related art, the reinforcing stylet resides substantially over the entire inflation lumen. Accordingly, the catheter shaft must have a large diameter in order to enhance the responsiveness for expansion and contraction of the balloon. However, in the present aspect, since the length of the core wire 31 extending inside the proximal-end shaft 23 is small, the responsiveness for the expansion and contraction of the balloon 21 is not impaired. Moreover, since the diameter is smaller, the maneuverability of the balloon catheter can be dramatically improved.

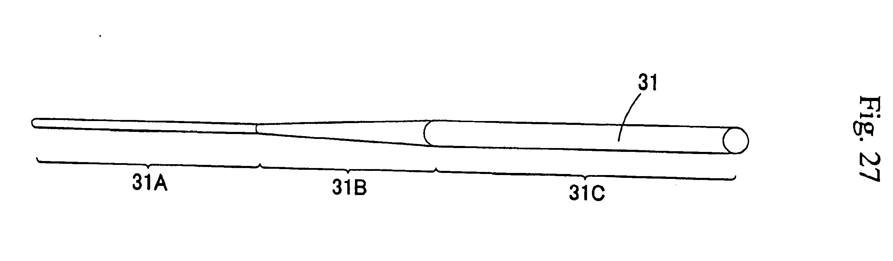

The core wire 31 enhances the maneuverability during pushing the balloon catheter from outside the body along the guidewire and prevents kinking (breakage) of the balloon catheter. In order to achieve such functions, the distribution of the rigidity in the longitudinal direction of the balloon catheter must be continuous. In particular, part of the core wire 31 located inside the proximal-end segment 22B should have a tapered shape in which the outer diameter of the core wire 31 decreases toward the distal end so that the continuous rigidity distribution can be realized. In one example of the core wire 31 shown in Fig. 27, a core-wire intermediate segment 31B preferably resides inside the proximal-end segment 22B of the distal-end shaft.

The proximal-end opening 25B of the guidewire lumen 25 is distant in the distal-end direction from the proximal-end shaft 23 by the length of the proximal-end segment 22B. In such a case, the length of the distal-end shaft 22, i.e., the lengths of the distal-end segment 22A and the proximal-end segment 22B, are not particularly limited and may be adjusted according to the location in which the balloon catheter is employed. For example, when the aspect is applied to a percutaneous transluminal coronary angioplasty (PTCA) catheter, the length of the distal-end shaft 22 is 100 to 600 mm, preferably 200 to 500 mm, the length of the distal-end segment 22A of the distal-end shaft (approximately equal to the length of the guidewire lumen 25) is 50 to 450 mm, and preferably 150 to 350 mm, and the length of the proximal-end segment 22B of the distal-end shaft is 50 to 300 mm, and preferably 50 to 200 mm. The lengths of the individual segments may be adjusted within the above-described ranges according to the location where the balloon catheter is employed.

The outer diameters and the inner diameters of the distal-end shaft 22, i.e., the distal-end segment 22A and the proximal-end segment 22B, and the proximal-end shaft 23 are not particularly limited. For every component, smaller the outer diameter, the better the insertion characteristics of the balloon catheter into the narrowed areas. However, the outer and inner diameters should be selected while considering the cross-section area in the diameter direction of the inflation lumen 26, which strongly affects the responsiveness for the expansion and contraction of the balloon 21, the pressure resistance of the distal-end shaft 22, and the size of the core wire 31. For example, regarding the outer diameter, the outer diameter of the distal-end segment 22A or the proximal-end segment 22B of the PTCA balloon catheter is 0.75 to 1.10 mm, and preferably 0.80 to 0.95 mm. The outer diameter of the proximal-end shaft 23 is 0.55 mm to 0.95 mm, and preferably 0.60 to 0.85 mm.

The shape and the dimensions of the core wire 31 may be determined based on the size and the material of the distal-end shaft 22 and the proximal-end shaft 23 and the intended use. Fig. 27 shows an example of the shape of the core wire 31. However, this example does not limit the shape and the size of the core wire 31. In the example shown in Fig. 27, the core-wire intermediate segment 31B having a tapered shape with a decreasing diameter toward the distal end is preferably located inside the proximal-end segment 22B of the distal-end shaft. A core wire distal-end segment 31A preferably resides inside the distal-end segment 22A of the distal-end shaft, the proximal end of the core-wire distal-end segment 31A is preferably affixed to the region near the proximal-end opening 25B of the guidewire lumen 25, and a part of a core-wire proximal-end segment 31C preferably resides inside the proximal-end shaft 23. In the PTCA balloon catheter, the core wire 31 has an outer diameter of 0.08 mm to 0.30 mm, and preferably 0.10 mm to 0.25 mm, and a length of 20 mm to 200 mm, and preferably 30 mm to 150 mm; the core-wire proximal-end segment 31C has a diameter of 0.20 mm to 0.50 mm, and preferably 0.25 mm to 0.40 mm, and a length of 20 mm to 200 mm, and preferably 30 mm to 150 mm; and the core-wire intermediate segment 31B has a length of 10 mm to 100 mm, and preferably 20 mm to 80 mm. The outer diameter of the core-wire intermediate segment 31B may be same as those of the core-wire distal-end segment 31A and the core-wire proximal-end segment 31C.

The core wire 31 may be composed of any metal material. The material may be selected according to the size and the material of the distal-end shaft 22 and the proximal-end shaft 23 and the intended use of the balloon catheter. From the standpoint of the workability and safety in vivo, stainless steel is preferred. Moreover, the method for making the tapered segments such as core-wire intermediate segment 31B or narrow segments such as core-wire distal-end segment 31A in the core wire 31 is not particularly limited; a known technique such as centerless grinding may be suitably employed.

Examples of the method for making the expandable and contractible balloon 21 by adjusting the inner pressure include dip-molding and blow-molding. A suitable method may be selected according to the intended use of the balloon catheter. Blow molding is preferably employed in making a PTCA balloon catheter in order to obtain sufficient pressure resistance. An example of making the balloon 21 by blow-molding will now be described. First, a tubular parison having predetermined dimensions is prepared by extrusion molding or the like. The tubular parison is then placed in a die having the shape corresponding to the shape of the balloon and is stretched in the axis direction and the diameter direction in the biaxial stretching step to form the balloon 21 having the same shape as that of the die. Note that the biaxial stretching step may be performed with heating or two or more times. Moreover, the stretching in the axis direction may be performed at the same time with, before, or after the stretching in the diameter direction. Moreover, annealing may be performed to stabilize the shape and the dimensions of the balloon 21.

The balloon 21 has a straight tubular segment 21A, a tapered segment 21B at the front of the straight tubular segment 21A, a tapered segment 21C at the back of the straight tubular segment 21A, joint segments 21D continuously attached to the ends of the tapered segments 21B and 21C, respectively. The dimensions of the balloon 21 are determined according to the intended use of the balloon catheter. The outer diameter of the straight tubular segment 21A is 1.50 mm to 35.00 mm, and preferably 1.50 mm to 30.00 mm, the length of the straight tubular segment 21A is 8.00 mm to 80.00 mm, and preferably 9.00 mm to 60.00 mm after deployment by adjusting the internal pressure.

The tubular parison may be composed of any resin. Examples of the resin include, polyolefins, polyolefin elastomers, polyesters, polyester elastomers, polyamides, polyamide elastomers, polyurethanes, and polyurethane elastomers. Moreover, a blend material or multilayer material containing two or more of these resins may also be used.

The material of the distal-end shaft 22, i.e., the distal-end segment 22A and the proximal-end segment 22B, is not particularly limited. When the distal-end segment 22A is of the coaxial type, the inner tube 29 may be composed of polyolefin, a polyolefin elastomer, polyester, a polyester elastomer, polyamide, a polyamide elastomer, polyurethane, a polyurethane elastomer, or the like. However, since the inner wall of the inner tube 29 defines the guidewire lumen 25, polyethylene, in particular, high-density polyethylene, is preferred from the standpoint of the slidability of the guidewire. More preferably, at least part of the inner tube 29 has a multilayer structure having a high-density polyethylene innermost layer and an outermost layer composed of a material that can be melt-bonded with the balloon 21 or the outer tube 28. The multilayered portion becomes the core-wire affixation region 32, thereby easily realizing the present aspect. The inner wall of the inner tube 29 may be coated with silicon, polytetrafluoroethylene, or the like to enhance the slidability of the guidewire.

When the distal-end segment 22A of the distal-end shaft is of the coaxial type, the material of the outer tube 28 is not particularly limited. Examples of the material include polyolefins, polyolefin elastomers, polyesters, polyester elastomers, polyamides, polyamide elastomers, polyurethane, and polyurethane elastomers.

When the distal-end segment 22A of the distal-end shaft is of a biaxial type or any other type, the distal-end segment 22A may also be composed of the material of the inner tube 29 or the outer tube 28. The distal-end segment 22A may be a multilayer structure made by a known technique. Needless to say, the outer tube 28 that constitutes the proximal-end segment 22B may be composed of the above-described material. The material, the dimensions, and the like of the outer tube 28 that constitutes the distal-end segment 22A of the distal-end shaft and the outer tube 28 that constitutes the proximal-end segment 22B of the distal-end shaft may be selected based on the cross-sectional area of the inflation lumen 26 and the rigidity distribution of the balloon catheter.

The proximal-end shaft 23 does not limit the advantages of the present aspect as long as it is composed of metal. Although various types of metal may be used according to the size and material of the distal-end shaft 22, stainless steel is particularly preferred from the standpoint of workability and the safety in vivo. In order to effectively achieve the continuous rigidity distribution in the longitudinal direction of the balloon catheter, a spiral notch, groove, or slit may be formed at the distal-end portion of the proximal-end shaft 23. In this manner, the rigidity of the distal-end portion of the proximal-end shaft 23 can be made smaller than that of the proximal-end portion of the proximal-end shaft 23, thereby yielding continuous rigidity distribution.

Preferable examples of the material of the hub 24 include resins such as polycarbonate, polyamide, polyurethane, polysulfone, polyarylate, styrene-butadiene copolymers, and polyolefin.

The method for joining the balloon 21 with the distal-end shaft 22 is not particularly limited, and any known technique may be applied. Examples of the method include bonding using an adhesive and melt bonding. Melt bonding can be used where the balloon 21 and the distal-end shaft 22 are composed of materials that can melt-bond. Moreover, when an adhesive is used, the composition, the chemical structure, and the cure type of the adhesive are not limited. In particular, from the standpoint of the composition and the chemical structure of the adhesive, adhesives of a urethane type, a silicone type, an epoxy type, a cyanoacrylate type, or the like may be suitable used. Regarding the cure method, an adhesive of a two-liquid mix type, a UV-curable type, a moisture absorption curing type, a heat curing type, or the like may be employed. It is preferable to use an adhesive such that the rigidity of the joined portions between the balloon 21 and the distal-end shaft 22 does not make the rigidity discontinuous over the joined portions. It is possible to select an adhesive based on the material, the size, and the rigidity of the balloon 21 and the distal-end shaft 22. In order to reduce the diameter of the joined portions, the joined portions may be heated. Moreover, when one or both of the balloon 21 and the distal-end shaft 22 is composed of a nonadhesive material such as polyolefin, the joined portions may be plasma-treated with oxygen gas or the like to improve the adhesiveness.

The distal-end shaft 22 is composed of resin and the proximal-end shaft 23 is a metal tube. Thus, the method of joining the distal-end shaft 22 to the proximal-end shaft 23 is limited to adhesive bonding. However, as described above, it is obvious that the advantages of the present aspect are not adversely affected by the composition, chemical structure, or cure method of the adhesive. Moreover, a suitable method, such as heating, may be employed to narrow the joined portions after application of the adhesive in order to reduce the diameter of the joined portions of the distal-end shaft 22 and the proximal-end shaft 23.

In order to improve the visibility of the balloon 21 during operation of the balloon catheter and to locate the balloon 21, a radiopaque marker 27 may be mounted on the outer wall of the part of the guidewire lumen 25 inside the balloon 21. The radiopaque marker 27 may be composed of a radiopaque material of any type, e.g., metal or resin. The radiopaque marker 27 may be anywhere as long as it is inside the balloon 21. The number of the radiopaque marker 27 is also not limited, and should be selected according to the intended use of the balloon catheter.

The outer wall of the balloon catheter may be provided with hydrophilic coating in order to facilitate insertion of the guide catheter into a blood vessel or the guide catheter. In particular, the areas that make contact with blood, e.g., the outer wall of the distal-end shaft 22, the outer wall of the proximal-end shaft 23, and the outer surface of the balloon 21, may be provided with hydrophilic coating that exhibits lubricity when the coating comes into contact with blood. The area on which the hydrophilic coating is provided and the length of the coated area may be determined according to the intended use of the balloon catheter. The type of the hydrophilic coating does not limit the effect of the present aspect. Hydrophilic polymers such as poly(2-hydroxyethylmethacrylate), polyacrylamide, polyvinylpyrrolidone, and the like may be suitably used. The coating method is not particularly limited.

The outer surface of the balloon 21 may be provided with hydrophobic coating to prevent slipping of the balloon 21 during expansion of the balloon 21 under particular intended uses of the balloon catheter. No limit is imposed on the type of the hydrophobic coating; a hydrophobic polymers such as silicon can be suitably used.

### Industrial Applicability

As is described above, the invention provides a stent delivery catheter that can place a stent in a tortuous narrowed area with good maneuverability while preventing falling or displacement of the stent.

As is described above, a second aspect provides a stent delivery catheter that can prevent a stent from falling or moving in the course of inserting the catheter to a narrowed area. Moreover, since no additional components are necessary to prevent displacement, the number of components or manufacturing steps does not increase. Thus, the manufacturing process can be streamlined without complication.

As is described above, a third aspect provides a balloon catheter having improved maneuverability in inserting the balloon catheter along the guidewire from outside the body. Moreover, an RX-type balloon catheter having enhanced responsiveness for balloon expansion and contraction can be provided without complicating the process and increasing the manufacturing cost. The balloon catheter of the third aspect can be applied to wide medical uses, such as percutaneous angioplasty (percutaneous transluminal angioplasty (PTA), percutaneous transluminal coronary angioplasty (PTCA), or the like), e.g., peripheral angioplasty, coronary vein angioplasty, and valvoplasty.

## Claims

1. A stent delivery catheter for delivering a stent for treating stenosis in a body to a narrowed area, the stent delivery catheter comprising: a distal end; and a proximal end, wherein the proximal end comprises a hub (9) having a port for supplying a pressure fluid, and the distal end comprises a collapsible balloon (1) in a collapsed state and the stent (11) in an undeployed state, the stent (11) being mounted on the outer surface of the collapsed balloon, the balloon (1) having frustoconical tapered segments (1B) (1C) and a cylindrical straight tubular segment (1A), wherein an inner tube (3) for defining a guidewire lumen (5) extends into the interior of the balloon (1); and displacement prevention mechanisms (8) for preventing the stent (11) from moving in the longitudinal direction of the stent delivery catheter are affixed to the inner surface of the balloon (1) only.

2. The stent delivery catheter according to claim 1, wherein the displacement prevention mechanisms (8) are disposed at a distal end and a proximal end of the balloon (1).

3. The stent delivery catheter according to claim 1 or 2, wherein each displacement prevention mechanism comprises a tubular member.

4. The stent delivery catheter according to one of claims 1 to 3, wherein the displacement prevention mechanisms (8) lie only in the tapered segments of the balloon (1) and do not extend to the interior of the balloon (1).

5. The stent delivery catheter according to claim 4, wherein 1 ≤ (D2/D1), wherein D1 is the outer diameter of the displacement prevention mechanism (8) at the portion affixed to the balloon (1), and D2 is the outer diameter of the displacement prevention mechanism (8) at the portion extending into the interior of the balloon (1).

6. The stent delivery catheter according to claim 4, wherein 1 ≤ (D2/D1) ≤ 2, wherein D1 is the outer diameter of the displacement prevention mechanism (8) at the portion affixed to the balloon (1), and D2 is the outer diameter of the displacement prevention mechanism (8) at the portion extending into the interior of the balloon (1).

7. The stent delivery catheter according to one of claims 1 to 6, wherein each displacement prevention mechanism (8) comprises a material that can be melt-bonded with the balloon (1).

8. The stent delivery catheter according to claim 7, wherein the displacement prevention mechanism (8) comprises polyamide or a polyamide elastomer, and the balloon comprises a polyamide elastomer or a blend material containing polyamide elastomers.

9. The stent delivery catheter according to claim 7, wherein the displacement prevention mechanism (8) comprises polyester or a polyester elastomer, and the balloon comprises a polyester elastomer or a blend material containing polyester elastomers.

10. The stent delivery catheter according to one of claims 1 to 9, wherein a radiopaque marker (4) is mounted on the displacement prevention mechanism (8).

11. A balloon catheter using the stent delivery catheter according to one of claims 1 to 10, the balloon catheter comprising: a proximal-end shaft comprising a metal tube having a distal-end segment and a proximal-end segment; a distal-end shaft comprising a resin tube, a balloon (1) expandable and collapsible by adjusting the pressure of the interior; a guidewire lumen (5) that can accommodate a guidewire, the guidewire lumen (5) having a distal-end opening and a proximal-end opening; and an inflation lumen (6) for supplying a pressure fluid into the balloon (1), the guidewire lumen (5) extending in the balloon catheter distal-end direction over the interior of the balloon (1) so as to protrude from the balloon (1), the distal-end opening of the guidewire lumen (5) being formed in the front-most tip of the balloon catheter, the proximal-end opening of the guidewire lumen (5) being formed in the distal-end shaft, the distal end of the proximal-end shaft being joined with the proximal end of the distal-end shaft, and the distal end of the distal-end shaft being joined with the balloon, wherein a core wire for adjusting the flexibility of the distal-end shaft is disposed inside the inflation lumen (6) so that the portion extending from the proximal-end opening of the distal-end shaft to the proximal end of the distal-end shaft is harder than the portion extended from the proximal-end opening of the distal-end shaft to the distal end of the distal-end shaft and is softer than the proximal-end shaft, and wherein the core wire is affixed to the distal-end shaft only at the region near the proximal-end opening of the guidewire lumen.

12. The balloon catheter according to claim 11, wherein, the portion of the core wire in the region affixed to the distal-end shaft is wrapped with a resin layer that can melt-bond with the inner wall of the distal-end shaft for defining the inflation lumen (6).

13. The balloon catheter according to claim 11 or 12, wherein the distal end of the core wire is located at a position a predetermined distance away from the proximal-end opening of the guidewire lumen (5) in the distal-end direction.

14. The balloon catheter according to one of claims 11 to 13, wherein the proximal end of the core wire is located inside the proximal-end shaft but does not extend up to the proximal end of the proximal-end shaft.

15. The balloon catheter according to one of claims 11 to 14, wherein the outer diameter of at least part of the core wire in the region that extends in the proximal-end direction from the proximal-end opening of the guidewire lumen (5) in the distal-end shaft decreases toward the distal end of the core wire to form a tapered shape.

## Patentansprüche

1. Stent-Abgabekatheter zur Abgabe eines Stents zur Behandlung von Stenose in einem Körper an einen verengten Bereich, wobei der Stent-Abgabekatheter folgendes umfasst: ein distales Ende und ein proximales Ende, wobei das proximale Ende eine Nabe (9) mit einer Öffnung zum Zuführen eines Druckfluides umfasst, und das distale Ende einen faltbaren Ballon (1) in einem gefalteten Zustand und den Stent (11) in einem nicht entfalteten Zustand umfasst, wobei der Stent (11) auf der Außenfläche des gefalteten Ballons befestigt ist, wobei der Ballon (1) kegelstumpfförmige sich verjüngende Abschnitte (1B), (1C) und ein zylindrisches gerades rohrförmiges Segment (1A) aufweist, wobei sich ein Innenrohr (3) zur Definition eines FührungsdrahtLumens (5) in das Innere des Ballons (1) erstreckt; und Verschiebungsverhinderungsmechanismen (8), um zu verhindern, dass sich der Stent (11) in Längsrichtung des Stent-Abgabekatheters bewegt, nur an der Innenfläche des Ballons (1) befestigt sind.

2. Stent-Abgabekatheter nach Anspruch 1, wobei die Verschiebungsverhinderungsmechanismen (8) an einem distalen Ende und einem proximalen Ende des Ballons (1) angeordnet sind.

3. Stent-Abgabekatheter nach Anspruch 1 oder 2, wobei jeder Verschiebungsverhinderungsmechanismus ein rohrförmiges Element umfasst.

4. Stent-Abgabekatheter nach einem der Ansprüche 1 bis 3, wobei die Verschiebungsverhinderungsmechanismen (8) nur in den sich verjüngenden Abschnitten des Ballons (1) liegen und sich nicht in das Innere des Ballons (1) erstrecken.

5. Stent-Abgabekatheter nach Anspruch 4, wobei 1≤(D2/D1) gilt, wobei D1 der Außendurchmesser des Verschiebungsverhinderungsmechanismus (8) an dem Teil ist, der an dem Ballon (1) befestigt ist, und D2 der Außendurchmesser des Verschiebungsverhinderungsmechanismus (8) an dem Teil ist, der sich in das Innere des Ballons (1) erstreckt.

6. Stent-Abgabekatheter nach Anspruch 4, wobei 1≤(D2/D1)≤2 gilt, wobei D1 der Außendurchmesser des Verschiebungsverhinderungsmechanismus (8) an dem Teil ist, der an dem Ballon (1) befestigt ist, und D2 der Außendurchmesser des Verschiebungsverhinderungsmechanismus (8) an dem Teil ist, der sich in das Innere des Ballons (1) erstreckt.

7. Stent-Abgabekatheter nach einem der Ansprüche 1 bis 6, wobei jeder Verschiebungsverhinderungsmechanismus (8) ein Material umfasst, das mit dem Ballon (1) schmelzgebondet werden kann.

8. Stent-Abgabekatheter nach Anspruch 7, wobei der Verschiebungsverhinderungsmechanismus (8) ein Polyamid oder ein Polyamid-Elastomer umfasst und der Ballon ein Polyamid-Elastomer oder ein Materialgemisch umfasst, das Polyamid-Elastomere enthält.

9. Stent-Abgabekatheter nach Anspruch 7, wobei der Verschiebungsverhinderungsmechanismus (8) ein Polyester oder ein Polyester-Elastomer umfasst und der Ballon ein Polyester-Elastomer oder ein Materialgemisch, das Polyester-Elastomere enthält, umfasst.

10. Stent-Abgabekatheter nach einem der Ansprüche 1 bis 9, wobei ein radioopaker Marker (4) auf dem Verschiebungsverhinderungsmechanismus (8) befestigt ist.

11. Ballonkatheter unter Verwendung des Stent-Abgabekatheters nach einem der Ansprüche 1 bis 10, wobei der Ballon-Abgabekatheter folgendes umfasst:
einen proximalen Endschaft, der ein Metallrohr mit einem distalen Endabschnitt und einem proximalen Endabschnitt umfasst; einen distalen Endschaft, der ein Harzrohr umfasst; einen durch Einstellen des Drucks des Inneren auf- und zusammenfaltbaren Ballon (1); ein Führungsdrahtlumen (5), das einen Führungsdraht aufnehmen kann, wobei das Führungsdrahtlumen (5) eine distale Endöffnung und eine proximale Endöffnung aufweist; und ein Inflationslumen (6) zum Zuführen eines Druckfluids in den Ballon (1), wobei sich das Führungsdrahtlumen (5) in der Richtung des distalen Endes des Ballon-Katheters durch das Innere des Ballons (1) erstreckt, so dass es über den Ballon (1) hinausragt, wobei die distale Endöffnung des Führungsdrahtlumens (5) in der vordersten Spitze des Ballonkatheters ausgebildet ist, wobei die proximale Endöffnung des Führungsdrahtlumens (5) in dem distalen Endschaft ausgebildet ist, wobei das distale Ende des proximalen Endschafts mit dem proximalen Ende des distalen Endschafts verbunden ist, und das distale Ende des distalen Endschafts mit dem Ballon verbunden ist, wobei ein Kerndraht zum Einstellen der Biegsamkeit des distalen Endschafts im Inneren des Inflationslumens (6) angeordnet ist, so das der Teil, der sich von der proximalen Endöfnung des distalen Endschafts zum proximalen Ende des distalen Endschafts erstreckt, härter ist als der Teil, der sich von der proximalen Endöffnung des distalen Endschafts zum distalen Ende des distalen Endschafts erstreckt und weicher ist als der proximale Endschaft, und wobei der Kerndraht nur in dem Bereich in der Nähe der proximalen Endöffnung des Führungsdrahtlumens am distalen Endschaft befestigt ist.

12. Ballonkatheter nach Anspruch 11, wobei der Teil des Kerndrahts in dem Bereich, der am distalen Endschaft befestigt ist, mit einer Harzschicht umwickelt ist, die mit der Innenwand des distalen Endschafts zur Festlegung des Inflationsvolumens (6) schmelzgebondet werden kann.

13. Ballonkatheter nach Anspruch 11 oder 12, wobei das distale Ende des Kerndrahts an einer Position in einem vorbestimmten Abstand von der proximalen Endöffnung des Führungsdrahtlumens (5) in der distalen Endrichtung angeordnet ist.

14. Ballonkatheter nach einem der Ansprüche 11 bis 13, wobei das proximale Ende des Kerndrahts im Inneren des proximalen Endschafts angeordnet ist, aber sich nicht bis zum proximalen Ende des proximalen Endschafts erstreckt.

15. Ballonkatheter nach einem der Ansprüche 11 bis 14, wobei der Außendurchmesser von mindestens einem Teil des Kerndrahts in dem Bereich, der sich in proximaler Endrichtung von der proximalen Endöffnung des Führungsdrahtlumens (5) in dem distalen Endschaft erstreckt, in Richtung des distalen Endes des Kerndrahts unter Bildung einer sich verjüngenden Form abnimmt.

## Revendications

1. Cathéter de pose d'endoprothèse pour positionner une endoprothèse pour le traitement de la sténose dans un corps dans une zone resserrée, le cathéter de pose pour endoprothèse comprenant : un bout distal ; et un bout proximal, dans lequel le bout proximal comprend un moyeu (9) ayant un orifice pour fournir un fluid de pression, et le bout distal comprend un ballon pliable (1) dans un état plié et l'endoprothèse (11) dans un état non déployé, l'endoprothèse (11) étant montée sur la surface extérieure du ballon plié, le ballon (1) ayant des segments tronconiques fuselés (1B) (1C) et un segment droit cylindrique tubulaire (1A), dans lequel une tube intérieure (3) pour déterminer un lumen (5) de fil de guide s'étend vers l'intérieur du ballon (1) ; et des mécanismes de prévention de déplacement (8) pour prévenir que l'endoprothèse (11) se déplace vers la direction longitudinale du cathéter de pose d'endoprothèse sont uniquement fixés à la surface intérieure du ballon (1).

2. Cathéter de pose d'endoprothèse selon la revendication 1, dans lequel les mécanismes de prévention de déplacement (8) sont disposés à un bout distal et un bout proximal du ballon (1).

3. Cathéter de pose d'endoprothèse selon la revendication 1 ou 2, dans lequel chacun des mécanismes de prévention de déplacement comprend un élément tubulaire.

4. Cathéter de pose d'endoprothèse selon l'une des revendications 1 à 3, dans lequel les mécanismes de prévention de déplacement (8) sont exclusivement situés dans les segments fuselés du ballon (1) et ne s'étendent pas vers intérieure du ballon (1).

5. Cathéter de pose d'endoprothèse selon la revendication 4, dans lequel 1≤(D2/D1), dans lequel D1 est le diamètre extérieur du mécanisme de prévention de déplacement (8) à la partie qui est fixée au ballon (1), et D2 est le diamètre extérieure du mécanisme de prévention de déplacement (8) à la partie qui s'étend vers l'intérieure du ballon (1).

6. Cathéter de pose d'endoprothèse selon la revendication 4, dans lequel 1≤(D2/D1)≤2, dans lequel D1 est le diamètre extérieur du mécanisme de prévention de déplacement (8) à la partie qui est fixée au ballon (1), et D2 est le diamètre extérieure du mécanisme de prévention de déplacement (8) à la partie qui s'étend vers l'intérieure du ballon (1).

7. Cathéter de pose d'endoprothèse selon l'une des revendications 1 à 6, dans lequel chacun des mécanismes de prévention de déplacement (8) comprend un matériau qui peut être lié à la coulée avec le ballon (1).

8. Cathéter de pose d'endoprothèse selon la revendication 7, dans lequel le mécanisme de prévention de déplacement (8) comprend du polyamide ou un élastomère de polyamide ou un matériau mélangé contenant des élastomères de polyamide.

9. Cathéter de pose d'endoprothèse selon la revendication 7, dans lequel le mécanisme de prévention de déplacement (8) comprend du polyester ou un élastomère de polyester, et le ballon comprend un élastomère de polyester ou un matériau mélangé contenant des élastomères de polyester.

10. Cathéter de pose d'endoprothèse selon l'une des revendications 1 à 9, dans lequel un marqueur radiopaque (4) est monté sur le mécanisme de prévention de déplacement (8).

11. Cathéter de ballon utilisant le cathéter de pose d'endoprothèse selon l'une des revendications 1 à 10, le cathéter de pose d'endoprothèse comprenant : une tige de bout proximal comprenant une tube de métal ayant un segment de bout distal et un segment de bout proximal ; une tige de bout distal comprenant une tube de résine ; un ballon (1) expansible et pliable par réglage la pression de l'intérieur ; un lumen (5) de fil de guide qui peut contenir un fil de guide, le lumen (5) de fil de guide ayant une orifice de bout distal et une orifice de bout proximal ; et un lumen d'inflation (6) pour fournir un fluid de pression dans le ballon (1), le lumen (5) de fil de guide s'étendant vers la direction du bout distal du cathéter de ballon à travers l'intérieur du ballon (1) pour qu'il dépasse le ballon (1), l'orifice de bout distal du lumen (5) de fil de guide étant formée dans la pointe la plus avancée du cathéter de ballon, l'orifice de bout proximal du lumen (5) de fil de guide étant formée dans la tige de bout distal, le bout distal de la tige de bout proximal étant lié au bout proximal de la tige de bout distal, et le bout distal de la tige de bout distal étant lié au ballon, dans lequel un fil de noyau pour régler la flexibilité de la tige de bout distal est disposé à l'intérieur du lumen (6) d'inflation pour que la partie s'étendant de l'orifice de bout proximal de la tige de bout distal au bout proximal de la tige de bout distal soit plus dure que la partie s'étendant de la orifice de bout proximal de la tige de bout distal au bout distal de la tige de bout distal et est plus tendre que la tige de bout proximal, et dans lequel le fil de noyau est fixé à la tige de bout distal uniquement à la région près de l'orifice de bout proximal du lumen de fil de guide.

12. Cathéter de ballon selon la revendication 11, dans lequel la partie du fil de noyau dans la région qui est fixée à la tige de bout distal est enveloppée avec une couche de résine qui peut être liée à la coulée à la paroi intérieure de la tige de bout distal pour déterminer le lumen d'inflation (6).

13. Cathéter de ballon selon la revendication 11 ou 12, dans lequel le bout distal du fil de noyau est localisé à une position à une distance prédéterminé de l'orifice de bout proximal du lumen (5) de fil de guide dans la direction de bout distal.

14. Cathéter de ballon selon la revendication 11 à 13, dans lequel le bout proximal du fil de noyau est localisé à l'intérieure de la tige de bout proximal mais ne s'étend pas jusau'au bout proximal de la tige de bout proximal.

15. Cathéter de ballon selon la revendication 11 à 14, dans lequel le diamètre extérieur d'au moins une partie du fil de noyau dans la région qui s'étend en direction de bout proximal de l'orifice de bout proximal du lumen (5) de fil de guide dans la tige de bout distal diminue vers le bout distal du fil de noyau pour former une forme fuselée.
